# FASCICULE DE BREVET EUROPEEN

(11) **EP 1 769 011 B1**
(45) Date de publication et mention de la délivrance du brevet: **27.05.2009**
(21) Numéro de dépôt: 05786121.3
(22) Date de dépôt: 01.07.2005
(51) Int. Cl.: C08F 220/34

(54) **COPOLYMERES ETHYLENIQUES, COMPOSITIONS LES COMPRENANT ET PROCEDE DE TRAITEMENT**
ETHYLEN-COPOLYMERE, DIESE COPOLYMERE ENTHALTENDE ZUSAMMENSETZUNGEN UND BEHANDLUNGSVERFAHREN
ETHYLENE COPOLYMERS, COMPOSITIONS CONTAINING SAID COPOLYMERS AND TREATMENT METHOD

(30) Priorité: 02.07.2004 FR 0451411; 14.07.2004 US 587553 P
(43) Date de publication de la demande: 04.04.2007
(73) Titulaire: L'Oréal, 75008 Paris (FR)
(72) Inventeur: MOUGIN, Nathalie, 75011 Paris (FR); JEGOU, Gwenaëlle, F-93190 Livry Gargan (FR)
(74) Mandataire: Dodin, Catherine
(86) Numéro de dépôt international: PCT/FR2005/001699
(87) Numéro de publication internationale: WO 2006/013268

(56) Documents cités:
- EP-A- 0 372 546
- EP-A- 0 947 244
- FR-A- 2 419 947
- US-A- 5 608 021
- US-A1- 2004 052 746
- PATENT ABSTRACTS OF JAPAN vol. 1999, no. 12, 29 octobre 1999 (1999-10-29) & JP 11 181029 A (KAO CORP), 6 juillet 1999 (1999-07-06)

## Description

La présente invention a trait à de nouveaux polymères, à leur utilisation notamment en cosmétique, ainsi qu'aux compositions les comprenant.

Il est connu d'employer des polymères dans le domaine cosmétique, et notamment en capillaire, par exemple pour apporter de la tenue ou du coiffant à la chevelure. Dans le domaine des compositions capillaires dites "rincées", telles que les shampooings ou après-shampooings, on utilise notamment des polymères cationiques synthétiques, solubles dans l'eau, qui sont connus pour apporter une bonne cosmétique aux cheveux; toutefois, ces polymères n'apportent aucun effet de mise en forme des cheveux. Il en est de même avec les polymères dérivés naturels cationiques tels les guars modifiées qui apportent également un caractère cosmétique sans permettre une mise en forme. Dans le domaine des compositions rincées, il n'est pas connu de polymères apportant du coiffant et une cosmétique acceptable. Dans le domaine des compositions capillaires dites "non rincées", telles que les produits de coiffage de type laques, gels ou sprays coiffants, on est constamment à la recherche de polymères apportant des effets coiffants, et de la tenue à la chevelure. On connaît dans les produits de coiffage, des polymères à motifs amines, tels que les polymères à base de vinylpyrrolidone et de méthacrylate de diméthylaminoéthyle (polymères Gaffix). Les compositions obtenues présentent toutefois une tenue insuffisante dans le temps. On connaît également des polymères de type Luviquat, à base de vinylimidazole quaternisée et de vinylpyrrolidone, qui apportent de la douceur mais épaississent les compositions.

La présente invention a pour but de proposer des polymères capables d'apporter un réel effet coiffant tout en conservant une cosmétique acceptable aux compositions, et notamment des polymères peu visqueux qui ne modifient que faiblement la viscosité des compositions les comprenant.
Après de nombreuses recherches, la demanderesse a mis en évidence que l'utilisation de polymères comprenant entre autre des monomères du type (méth)acrylate de polyéthylèneglycol tels que définis ci-après, pouvait permettre la réalisation de compositions coiffantes ayant une cosmétique adéquate. Des polymères contenant des motifs (méth)acrylate de polyéthylène glycol (MPEG) sont décrits dans l'art antérieur. Ainsi, il est connu par EP372546, des copolymères à base de MPEG et de monomères de type (méth)acrylamides d'alkyles en C1-C8, pouvant comprendre des monomères cationiques. Toutefois, ces polymères ne comprennent qu'une faible proportion de monomères cationiques, ce qui ne leur permet pas de générer des effets cosmétiques adéquats, notamment un dépôt sur le cheveu qui soit suffisant pour apporter les propriétés recherchées.
Il revient à la demanderesse d'avoir constaté qu'il était préférable d'utiliser des polymères comprenant des taux de charge cationique élevés pour obtenir un dépôt satisfaisant dudit polymère sur le cheveu.
Le document JP2002-322219 décrit des polymères contenant des motifs MPEG en association avec des monomères hydrophobes à base de polypropylène glycol (PPO) ou de polyoxyde de tétraméthylène, et des monomères cationiques. Or, on a constaté que ces polymères qui comprennent des monomères hydrophobes, ne permettent pas d'obtenir des propriétés cosmétiques satisfaisantes.
Il est également connu par le brevet JP2002-284627 une composition comprenant des polymères cationiques dans lequel les monomères de type PEG sont associés à des monomères comprenant des motifs amines quaternaires. Or, la présence de motifs quaternaires peut induire, au fur et à mesure des applications, un surcroît de dépôt qui peut nuire, dans certains cas, à la qualité cosmétique de la composition. Par ailleurs, ces polymères contiennent un taux de charge cationique faible, de l'ordre de 0,5 à 6%, qui ne permet pas une affinité optimale pour le cheveu.
On connaît encore par JP2003-055164 des polymères contenant des motifs du type MPEG; toutefois ces polymères sont réticulés, ce qui rend difficile le contrôle de leur synthèse.
Le document JP2000-302649 décrit une composition capillaire comprenant un polymère qui comprend des monomères cationiques ou amphotères, des monomères à groupement polyéther, notamment de type PEG ou PPO, ainsi que des monomères optionnels qui peuvent être principalement hydrophobes (par exemple méthacrylate de stéaryle).
Il est également connu par le brevet JP07-285831, des compositions capillaires comprenant un polymère qui comprend des monomères de type MPEG en association avec des monomères ioniques, cationiques ou amphotères, et des monomères additionnels de type (méth)acrylates d'alkyle en C1-C24, principalement hydrophobes.
Toutefois, la présence de comonomères hydrophobes, par exemple de type acrylate de butyle ou de stéaryle, ne permet pas d'obtenir des propriétés cosmétiques adéquates, notamment ne permettent pas d'obtenir un bon démêlage des cheveux mouillés, juste après le shampoing.
On connaît encore la demande WO03/075867 qui décrit des copolymères blocs linéaires comportant une séquence poly(alkylène glycol) encadrée par deux séquences éthyléniques. Ces polymères présentent le défaut d'avoir une séquence centrale de type poly(alkylène glycol) de masse élevée qui confère au polymère une forte cristallinité, ce qui peut conduire à des produits opaques et/ou présentant un caractère gras.

La demanderesse a mis en évidence de nouveaux polymères permettant d'apporter un effet coiffant et conditionneur aux produits cosmétiques capillaires.
De manière surprenante, les polymères selon l'invention possèdent des propriétés cosmétiques intéressantes, par exemple lors de l'application dans une formulation de type shampooing; on a en effet constaté que les cheveux se démêlent facilement lors du shampooing, et qu'ils présentent de la douceur; après séchage, les compositions selon l'invention permettent également, une fois la chevelure séchée, une mise en forme des cheveux particulièrement intéressante.
Sans être tenu par la présente explication, on peut penser que ceci peut notamment être dû à la présence de motifs (méth)acrylate de PEG (MPEG) au sein de la chaîne de polymère, motifs qui contribuent en grande part à l'effet obtenu. On a en effet constaté que cet effet n'était pas obtenu avec un simple mélange de polymère cationique et de polymère de type PEG.

Un objet de la présente invention est donc un copolymère éthylénique constitué essentiellement, en % en poids par rapport au poids total du polymère:
- a) de 10% inclus à 60% exclus en poids d'au moins un monomère de formule (I) telle que définie ci-après, et/ou ses sels;
- b) de 40% exclus à 90% inclus en poids d'au moins un monomère "essentiellement cationique" et/ou ses sels, choisi parmi :
   - (i) un ou plusieurs monomères cationiques de formule (IIa),
   - (ii) un ou plusieurs monomères amphotères de formules (IIc) et (IId), et
   - (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId);
ainsi que les sels dudit copolymère.

Un autre objet de l'invention est une composition comprenant, dans un milieu physiologiquement acceptable, au moins un tel copolymère.

La présente invention a pour avantage de proposer des polymères généralement véhiculables dans l'eau, c'est-à-dire solubles ou dispersibles dans l'eau, ce qui permet de les employer de manière avantageuse dans des compostions cosmétiques, notamment de soin de la peau, ou capillaires, généralement à base aqueuse.
Par hydrosoluble ou soluble dans l'eau, on entend que le polymère forme une solution limpide dans l'eau, à raison d'au moins 5% en poids, à 25°C.
Par hydrodispersible ou dispersible dans l'eau, on entend que le polymère forme dans l'eau, à une concentration de 5 % en poids, à 25°C, une suspension ou dispersion stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Dans la suite de la présente description, on entendra par 'radical cyclique' un radical monocyclique ou polycyclique, qui peut se présenter lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).
On entendra par'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

Le copolymère éthylénique selon l'invention comprend donc au moins un monomère de formule (I), qui peut être présent seul ou en mélange, : 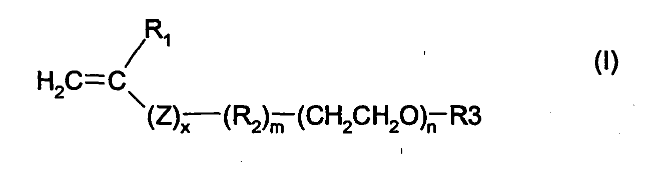 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO- , - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P; et leurs sels.

Notamment, R1 peut représenter un radical méthyle, éthyle, propyle, butyle. De préférence, R1 représente l'hydrogène ou un radical méthyle.

De préférence, Z représente COO ou CONH.

De préférence, x est égal à 1.

Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle).
Notamment R2 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule : 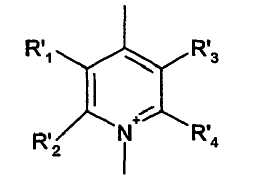 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH- ;-CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

De préférence, n est compris entre 5 et 200 inclus, et encore mieux entre 7 et 100 inclus, voire entre 9 et 50 inclus.

De préférence, R3 est un atome d'hydrogène; un radical benzyle ou phényle éventuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P.
Ces radicaux benzyle, phényle ou alkyle peuvent comprendre notamment une fonction choisie parmi les fonctions suivantes:

| | | | | | |
|---|---|---|---|---|---|
| Succinimido | Glutarate-succinimido | | | | Glutarate |
| 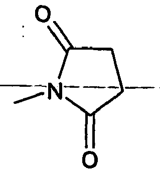 | 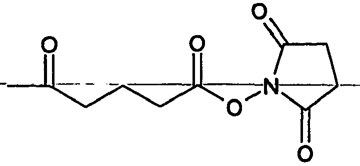 | | | | 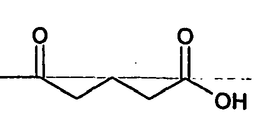 |
| maléimido | | Mésityle | | | Benzoate |
| 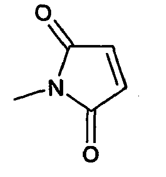 | | 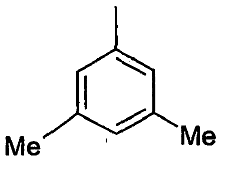 | | | 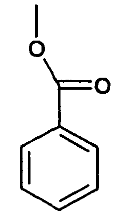 |
| Tosyle | | Triéthoxysilane | | | Phtalimide |
| 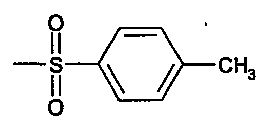 | | 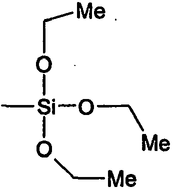 | | | 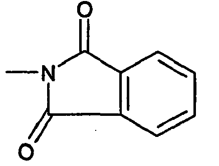 |
| Thioester | | Benzotriazole carbonate | | | Butyraldéhyde |
| 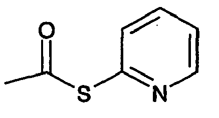 | | 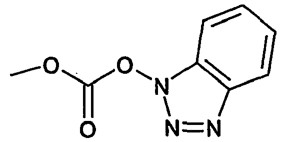 | | | 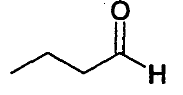 |
| Acétaldéhyde diéthylacétal | | | Biotine | | |
| 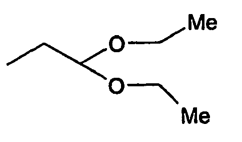 | | | 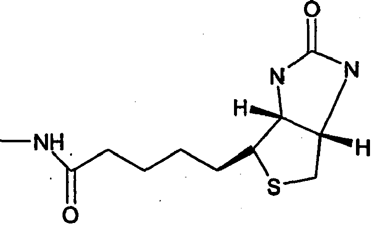 | | |
| Phospholipide | | | | Succinate N-hydroxysuccinimide | |
| 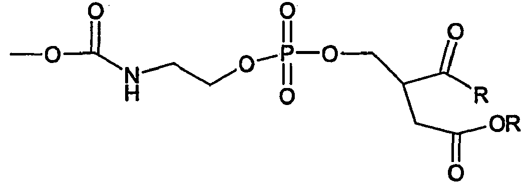 | | | | 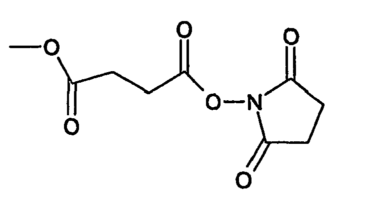 | |
| avec R = alkyle en C12-C18, et notamment lauryle, myristyle, palmityle, stéaryle, oléyle ou linoléyle | | | | | |

ou encore choisie parmi -SO₃H, -COOH, -PO₄, -NR5R6 ou -N⁺R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore portant des groupements protecteurs tels que le t-butyloxycarbonyle (aussi appelé BOC) ou le 9-fluorenylmethoxycarbonyle (aussi appelé FmoC).

Parmi les radicaux R3, on peut citer les chaînes méthyle, éthyle, propyle, benzyle, éthylhexyle, lauryle, stéaryle, béhényle (-(CH₂)₂₁-CH₃), et également les chaînes alkyles fluorées telles que par exemple heptadecafluorooctyl sulfonyl amino éthyle CF₃-(CF₂)₇-SO₂-N(C₂H₅)-CH₂-CH₂; ou encore les chaînes -CH₂-CH₂-CN, succinimido, maléimido, mésityle, tosyle, triéthoxysilane ou phtalimide.

Les motifs amines et/ou les groupes anioniques du monomère de formule (I) peuvent éventuellement être neutralisés, notamment de la manière décrite plus loin.

Parmi les monomères de formule (I) particulièrement préférés, on peut citer :
- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle;
- le monomère suivant : 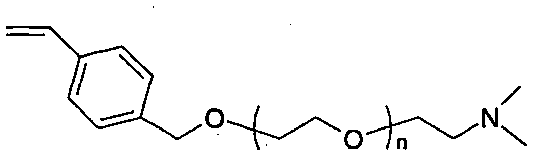 dans lequel n est de préférence compris entre 3 et 100 inclus, notamment 5 à 50 inclus, voire 7 à 30 inclus.

Les monomères de formule (I) tout particulièrement préférés sont choisis parmi les (méth)acrylates de poly(éthylène glycol) et les (méth)acrylates de méthyl-poly(éthylène glycol), de préférence ceux ayant un poids moléculaire compris entre 350 et 13 000 g/mol., notamment entre 500 et 8000 g/mol.
Tout particulièrement, on préfère les (méth)acrylates de poly(éthylène glycol) et en particulier ceux ayant un poids moléculaire entre 350 et 13 000 g/mol., notamment entre 500 et 8000 g/mol.

Des exemples de monomères commerciaux sont :
- le CD 350 (méthacrylate de méthoxy-poly(éthylène glycol 350) et le CD 550 (méthacrylate de méthoxy-poly(éthylène glycol 550), fourni par SARTOMER Chemicals;
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W.
- les poly(éthylène glycol) monomethyl éther, mono(succinimidyl succinate) ester de poids moléculaire moyen 1900 ou 5000, de chez Polysciences;
- le méthacrylate de behenyl poly(éthylène-glycol PEG-25), disponible chez Rhodia, sous la dénomination SIPOMER BEM;
- les acrylates de poly(éthylène glycol) phényl éther de poids moléculaires moyens 236, 280 ou 324 disponibles chez Aldrich;
- le méthoxy polyéthylène glycol 5000 2-(vinyl sulfonyl) éthyl éther disponible commercialement chez Fluka;
- le méthacrylate de polyéthylène glycol éthyl éther disponible chez Aldrich ;
- les méthacrylates de polyéthylène glycol 8000, 4000, 2000 de Monomer & Polymer Dajac laboratories ;
- le polyéthylène glycol N-hydroxy succinimide vinyl sulfone disponible commercialement chez Nektar Molecule enginnering ( Shearwater).

De préférence, le monomère de formule (I) a un poids moléculaire compris entre 350 et 13 000 g/mol., notamment entre 500 et 8000 g/mol.

Le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids.

Le copolymère éthylénique selon l'invention comprend également au moins un monomère "essentiellement cationique", et/ou l'un de ses sels, choisi parmi :
- (i) un ou plusieurs monomères cationiques de formule (IIa),
- (ii) un ou plusieurs monomères amphotères de formules (IIc) et (IId), et
- (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId).

De préférence, le monomère "essentiellement cationique" est choisi parmi les monomères cationiques de formule (IIa) et les monomères amphotères de formule (IIc) ou (IId), préférentiellement parmi les monomères cationiques de formule (IIa).

Par monomère cationique, on entend un monomère comprenant des motifs capables de posséder une charge cationique dans le domaine de pH compris entre 3 et 12. Ces motifs ne possèdent pas obligatoirement une charge permanente quelque soit le pH. L'unité cationique n'a pas besoin d'être protonée à chacun des ces pH. 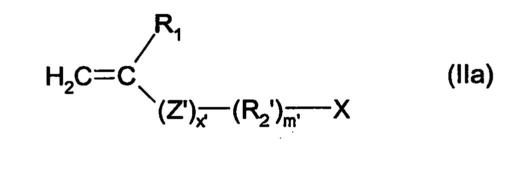 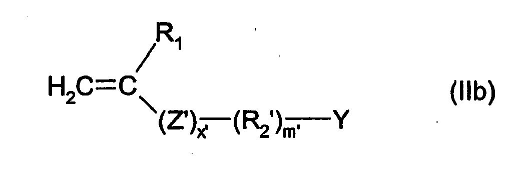 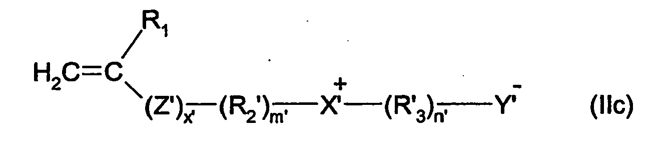 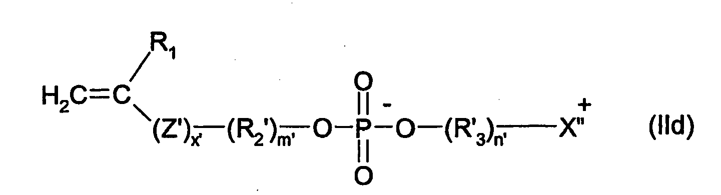 dans lesquelles :
- R1 est un atome d'hydrogène ou un radical hydrocarboné, linéaire ou ramifié, de type CₚH₂ₚ₊₁, avec p étant un entier compris entre 1 et 12, inclus; Notamment, R1 peut représenter un radical méthyle, éthyle, propyle, butyle. De préférence, R1 représente l'hydrogène ou un radical méthyle.
- Z' est un groupement divalent choisi parmi -COO-, -CONH-, -CONCH₃-, -OCO-
ou -O-, -SO₂- -CO-O-CO- ou -CO-CH₂-CO-;
De préférence, Z' est choisi parmi COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
   Dans le radical R'2, le ou les hétéroatomes, quand ils sont présents, peuvent être intercalés dans la chaîne dudit radical R'2, ou bien ledit radical R'2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle).
Notamment R'2 peut être :
- un radical alkylène tel que méthylène, éthylène, propylène, n-butylène, isobutylène, tertiobutylène, n-hexylène; n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 25 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;
- m' est 0 ou 1;
- X (dans la formule IIa) est un groupe guanidino, amidino, ou bien un groupe de formule -N(R₆)(R₇) ou -P(R₆)(R₇) ou -P⁺R₆R₇R₈, avec R6, R7 et R8 représentant, indépendamment l'un de l'autre, soit (i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; soit (iii) R6 et R7 peuvent former avec l'atome d'azote ou de phosphore, un premier cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N.
Par exemple, R6 et R7 peuvent être choisis parmi l'hydrogène, un groupement méthyle, éthyle, propyle, isopropyle, n-butyle, t-butyle, isobutyle, octyle, lauryle, stéaryle.
De préférence R6 et R7 sont choisis, indépendamment l'un de l'autre, parmi H, CH3 et C2H5.

De manière alternative, X peut représenter un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.
Par exemple, X peut constituer un cycle aromatique ou non comportant un groupement amine tertiaire ou peut représenter un hétérocycle aromatique ou non, contenant un azote tertiaire.
Parmi ces radicaux X préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

Les groupements guanidino et amidino sont respectivement de formule :

| | | | |
|---|---|---|---|
| Guanidino | 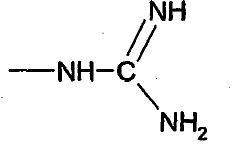 | Amidino | 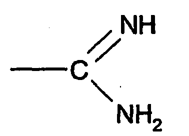 |

Les monomères de formule (IIa) peuvent être neutralisés par des agents neutralisants de nature chimique différentes, ainsi que cela est indiqué plus loin.

Parmi les monomères de formule (IIa) préférés, on peut citer :

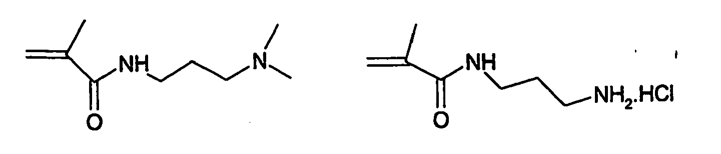

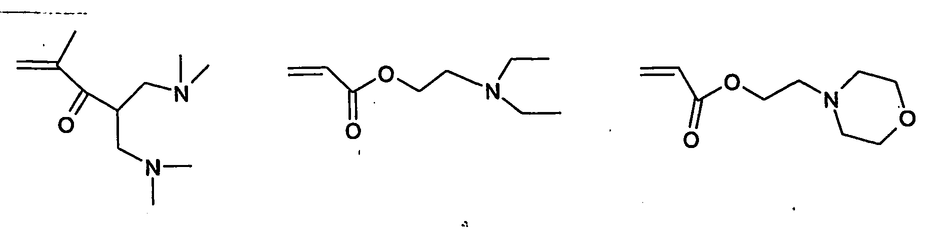

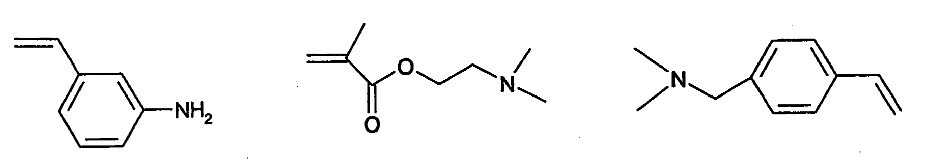

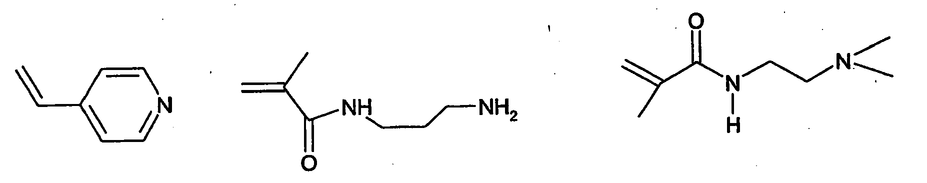

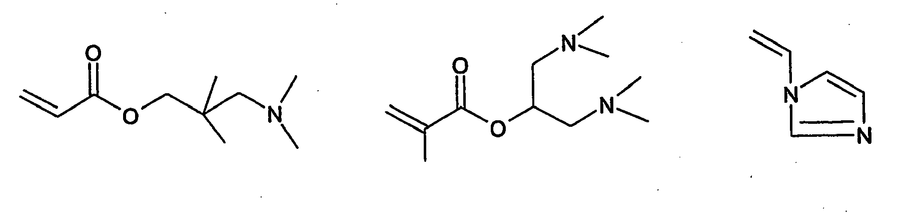

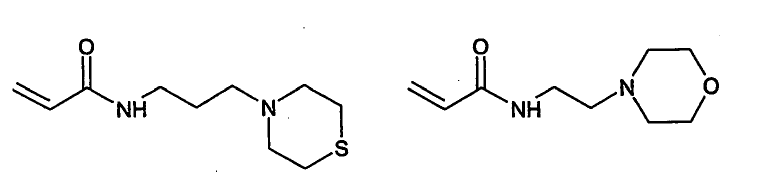

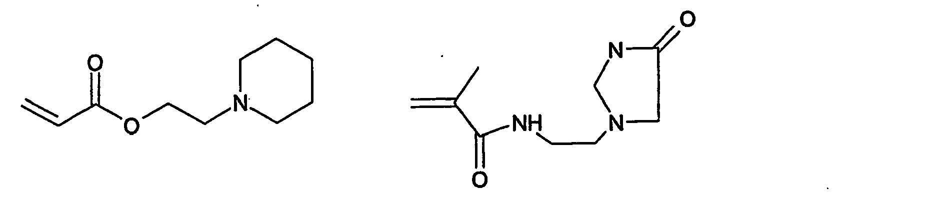

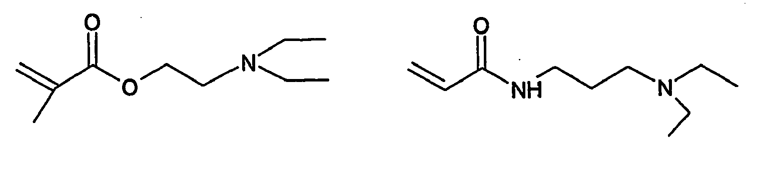

Parmi les monomères de formule (IIa) particulièrement préférés, on peut citer le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle.

Dans la formule (IIb), la signification des radicaux R1, Z', x', R'2 et m' est la même que celle donnée ci-dessus pour la formule (IIa).
Dans la formule (IIb), Y est un groupement choisi parmi -COOH, -SO₃H, -OSO₃H, -PO₃H₂ et -OPO₃H₂.

II est entendu que selon l'état de l'art, les groupes SO₄H₂ et PO₄H₂ sont liés à R'2 par l'atome d'oxygène tandis que les groupes SO₃H et PO₃H sont liés à R'2 respectivement via les atomes de S et P.

Les groupes anioniques des monomères de formule (IIb) peuvent éventuellement être neutralisés, notamment de la manière décrite plus loin.

Parmi les monomères anioniques préférés, on peut citer l'anhydride maléique et les monomères de formule (IIb) préférés suivants : l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.

Dans la formule (IIc), la signification des radicaux R1, Z', x', R'2 et m' est la même que celle donnée ci-dessus pour la formule (IIa). Les autres radicaux ont la signification suivante :
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.
   Par exemple, R6 et R7 peuvent être choisis parmi l'hydrogène, un groupement méthyle, éthyle, propyle, isopropyle, n-butyle, t-butyle ou isobutyle.
   Parmi les radicaux X'⁺ préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.
- Y'⁻ est un groupement choisi parmi -COO-, -SO₃⁻' -OSO₃⁻, -PO₃²- et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
   Dans le radical R'3, le ou les hétéroatomes, quand ils sont présents, peuvent être intercalés dans la chaîne dudit radical R'3, ou bien ledit radical R'3 peut être substitué par un ou plusieurs groupes les comprenant tels que hydroxy ou amino (NH₂, NHR' ou NR'R" avec R' et R" identiques ou différents représentant un alkyle linéaire ou ramifié en C1-C18, notamment méthyle ou éthyle).
Notamment R'3 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi O, N, S, F, Si et P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -[CH₂-CH₂-O]ₙ- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;
- n' est compris entre 1 et 100, de préférence 1 et 5 inclus.

Dans la formule (IId), la signification des radicaux R1, Z', x', R'2 et m' est la même que celle donnée ci-dessus pour la formule (IIa), et celle des radicaux R'3 et n' la même que celle donnée pour la formule (IIc).

Dans la formule (IId), X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N.
Par exemple, R6, R7 et R8 peuvent être choisis parmi l'hydrogène, un groupement méthyle, éthyle, propyle, isopropyle, n-butyle, t-butyle, isobutyle, octyle, lauryle ou stéaryle.

Parmi les radicaux X"⁺ préférés, on peut citer les radicaux triméthylammonium; triéthylammonium; N,N-diméthyl, N-octylammonium; N,N-diméthyl,N-laurylammonium.

Parmi les monomères de formule (IIc) ou (IId) préférés, on peut citer la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.

Lorsque le monomère 'essentiellement cationique' est choisi parmi les mélanges de monomères cationiques et/ou amphotères avec des monomères anioniques, lesdits monomères anioniques sont de préférence présents à raison de 5 à 40% en poids, par rapport au poids du mélange "cationiques et/ou amphotères + anioniques", notamment de 10 à 30% en poids, de préférence de 15 à 25% en poids.

Le monomère 'essentiellement cationique' est présent à raison de 40% exclus à 90% inclus, en poids par rapport au poids du polymère final, notamment de 45% inclus à 80% inclus en poids, de préférence de 50% inclus à 70% inclus en poids.

Le copolymère éthylénique est constitué essentiellement de monomère(s) de formule (I) et de monomère(s) essentiellement cationiques, selon l'invention, ce qui signifie qu'il ne comprend pas, de manière optionnelle, d'autres monomères que ceux mentionnés ci-dessus.

De préférence, le polymère selon l'invention comprend les monomères de formule (I) et les monomères "essentiellement cationiques" en un rapport pondéral pouvant aller de 60/40 à 40/60, avec une préférence pour un rapport 50/50.

Il est possible de neutraliser l'un et/ou l'autre des monomères ci-dessus avant polymérisation, ou bien de neutraliser le polymère une fois formé. De préférence, on neutralise le polymère après sa formation.

La neutralisation des groupes anioniques, qu'ils appartiennent au polymère et/ou aux monomères, notamment ceux de formule (I) et/ou (IIb), peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la dimethylamino2-propanol. On peut encore citer la lysine ou la 3-(diméthylamino)propylamine.

La neutralisation des motifs amines, appartenant au polymère et/ou aux monomères, notamment ceux de formule (I) et/ou (IIa), peut être effectuée par un acide minéral, tel que l'acide sulfurique, l'acide chlorhydrique, l'acide bromhydrique, l'acide iodhydrique, l'acide phosphorique, l'acide borique; ou bien par un acide organique, qui peut comporter un ou plusieurs groupes acide carboxylique, sulfonique ou phosphonique. II peut s'agir d'acides aliphatiques linéaires, ramifiés ou cycliques ou encore d'acides insaturés ou aromatiques. Ces acides peuvent comporter, en outre, un ou plusieurs hétéroatomes choisis parmi O, N, Si, F et P, par exemple sous la forme de groupes hydroxyle. On peut notamment citer l'acide propionique, l'acide acétique, l'acide téréphtalique, l'acide citrique, l'acide tartrique; la bétaïne HCl ou chlorhydrate de bétaïne de formule (CH₃)₃N+CH₂CO₂H.Cl-; l'acide gluconique, l'acide 2-éthylcaproïque, l'acide oléïque, l'acide béhénique et l'acide stéarique.

De manière avantageuse, le neutralisant des motifs amines peut être choisi parmi, seul ou en mélange, les agents neutralisants de type acides minéraux ou organiques, ayant un log P inférieur ou égal à 2, par exemple compris entre -8 et 2, de préférence compris entre -6 et 1, notamment compris entre -6 et 0.
Il peut également être choisi parmi, seul ou en mélange, les agents neutralisants acides minéraux ou organiques ayant un log P supérieur à 2, de préférence supérieur ou égal à 2,5, notamment supérieur à 3, et en particulier compris entre 3 et 15, voire entre 3,5 et 10.
Préférentiellement, il est choisi parmi les agents neutralisants de type acides minéraux ou organiques, ayant un log P inférieur ou égal à 2, par exemple compris entre -8 et 2, de préférence compris entre -6 et 1, notamment compris entre -6 et 0.

Les valeurs de log P sont connues et sont déterminées selon un test standard qui détermine la concentration du composé dans l'octanol-1 et l'eau.
Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional reference book, 1995). Il existe encore un site Internet qui fournit des valeurs estimées (adresse : http://esc.syrres.com/interkow/kowdemo.htm).

A titre d'information, on donne ci-après la valeur de log P de certains acides usuels :

| | |
|---|---|
| Acide sulfurique | -1.031+/-0.613 |
| Acide acétique | -0.285+/-0.184 |
| Acide propionique | 0.246+/-0.184 |
| Acide citrique | - 1.721 +/-0.396 |
| Acide gluconique | -3.175 +/- 0.852 |
| Acide borique | -0.292+/-0.753 |
| Acide phosphorique | -2.148+/-0.587 |
| Acide benzoïque | 1.895+/-0.206 |
| Acide stéarique | 8.216 +/-0.186 |
| Acide béhénique | 10.342+/-0.186 |
| Acide oléique | 7.698 +/- 0.199 |

De préférence, on peut utiliser comme agent neutralisant, l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement le chlorhydrate de bétaïne et/ou l'acide béhénique.

De préférence, le polymère selon l'invention est tel que :
- le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) et/ou les (méth)acrylates de méthyl-poly(éthylène glycol), de préférence ceux ayant un poids moléculaire compris entre 350 et 13 000 g/mol., notamment entre 500 et 8000 g/mol.; et/ou
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisi parmi, seul ou en mélange, les monomères de formule (IIb).

Tout particulièrement, on préfère les polymères dans lesquels:
- le monomère de formule (I), seul ou en mélange, est présent à raison de 10% inclus à 60% exclus, en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40% exclus à 90% inclus en poids par rapport au poids du polymère final notamment de 45% exclus à 80% inclus en poids, de préférence de 50% inclus à 70% inclus en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle;
   et de préférence en outre :
- le polymère est neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

Encore plus particulièrement, on préfère les polymères dans lesquels:
- le monomère de formule (I), seul ou en mélange, est présent à raison de 10 inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisi parmi, seul ou en mélange, le (méth)acrylamide de diméthylaminopropyle, et
- le polymère est neutralisé par un agent neutralisant choisi parmi l'acide béhénique et/ou le chlorhydrate de bétaïne.

Les polymères selon l'invention peuvent être préparés selon les méthodes usuelles de polymérisation radicalaire classique, bien connues de l'homme du métier, et telles que décrites par exemple dans l'ouvrage "Chimie et physicochimie des polymères" de Gnanou et al. (édition Dunod).
Notamment ces polymères peuvent être préparés par :
- polymérisation directe en solution dans l'eau avec pré-neutralisation ou non du motif cationique et/ou du motif anionique;
- polymérisation en émulsion dans l'eau avec pré-neutralisation ou non du motif cationique et/ou du motif anionique, avec utilisation d'un tensioactif;
- polymérisation dans un solvant organique, tel que l'éthanol ou la méthyléthylcétone, avec pré-neutralisation ou non du motif cationique et/ou du motif anionique, suivie d'une étape de mise en solution ou dispersion dans l'eau avec évaporation du solvant.
Ces polymérisations peuvent être effectuées en présence d'amorceur radicalaire notamment de type peroxyde (Trigonox 21 S : tert-butyl peroxy-2-ethylhexanoate)
ou azoïque (AIBN V50: 2,2'-azo-bis(2-amidinopropane) dihydrochlorure), ou persulfate de potassium ou d'ammonium, qui peut être présent à raison de 0,3 à 5% en poids par rapport au poids total des monomères.

Les polymères selon l'invention sont non réticulés. Ils se présentent sous forme de copolymères éthyléniques statistiques, de préférence filmogènes, d'un ou plusieurs monomères éthyléniques contenant des groupes PEG (les groupes PEG étant pendants le long du squelette) et d'un ou plusieurs monomères éthyléniques comportant des fonctions cationiques (amines neutralisées et non quaternaires) et/ou bétaïnes, de préférence monovalents.

Par polymère "éthylénique", on entend un polymère obtenu par polymérisation de monomères comprenant une insaturation éthylénique.
Par polymère "filmogène", on entend un polymère apte à former à lui seul ou en présence d'un agent auxiliaire de filmification, un film continu et adhérent sur un support, notamment sur les matières kératiniques.

Les copolymères selon l'invention présentent une masse moléculaire moyenne en poids (Mw) qui est de préférence comprise entre 500 et 5 000 000, notamment comprise entre 1 000 et 3 000 000 et plus préférentiellement comprise entre 2 000 et 2 000 000, voire 4 000 et 500 000, entre autre 7 000 et 400 000, encore mieux 20 000 et 350 000, et encore 150 000 et 300 000.
On détermine les masses molaires moyennes en poids (Mw) par chromatographie par perméation sur gel ou par diffusion de la lumière, selon l'accessibilité de la méthode (solubilité des polymères considérés).

Les polymères selon l'invention sont de préférence véhiculables en milieu aqueux, c'est-à-dire qu'ils sont de préférence hydrosolubles ou hydrodispersibles.
La mise en solution ou en dispersion dans l'eau peut être effectuée par solubilisation directe du polymère s'il est soluble, ou bien par neutralisation des motifs amines et/ou des motifs acides de façon à rendre le polymère soluble ou dispersible dans l'eau.
La mise en solution ou dispersion aqueuse peut également s'effectuer via une étape intermédiaire de solubilisation dans un solvant organique suivie de l'addition d'eau avant évaporation du solvant organique.

Par ailleurs, on a constaté que les polymères selon l'invention présentent avantageusement une viscosité dans l'eau adéquate avec les applications envisagées, qui peut être, par exemple comprise entre 1 et 1000 mPa.s, de préférence entre 1,5 et 750 mPa.s, et encore mieux entre 2 et 500 mPa.s.

La viscosité est mesurée à l'aide d'un viscosimètre BROOKFIELD, pour une solution à 15% en poids de polymère dans l'eau ou la méthyléthylcétone (solvant choisi en fonction de la solubilité du polymère et/ou de la méthode de polymérisation), à 25°C, avec un mobile de type aiguille (spindle) choisies parmi les modèles numéros 00 à 07 de Brookfield, de préférence le mobile no 1 ; pour un temps de mesure de 5 minutes, à une vitesse comprise entre 0,1 et 6 tours/minute. La viscosité est mesurée après complète dissolution du polymère dans l'eau ou la méthyléthylcétone.

En outre, les polymères selon l'invention peuvent présenter de préférence une température de transition vitreuse (Tg) comprise entre -150°C et 20°C, notamment -120°C et 10°C, mieux entre -100°C et 0°C; la Tg est mesurée selon la méthode donnée avant les exemples.
Les polymères selon l'invention peuvent présenter de préférence une température de fusion (Tf) comprise entre -100°C et 80°C, notamment entre -80°C et 50°C, mieux entre -70°C et 45°C, voire -10°C et 25°C.

En outre, les polymères selon l'invention présentent de préférence une reprise en eau comprise entre 3% et 150% en poids, de préférence entre 4% et 100% en poids, notamment entre 5% et 50% en poids, à 75% d'humidité relative (75%HR); la reprise en eau est mesurée selon la méthode donnée avant les exemples.
Ils peuvent également présenter une reprise en eau comprise entre 3% et 200% en poids, de préférence entre 2,5% et 150% en poids, notamment entre 3% et 100% en poids, à 85% d'humidité relative (85%HR).

Les polymères selon l'invention trouvent une application toute particulière dans le domaine de la cosmétique. Ils peuvent être présents dans la composition sous forme solubilisée, par exemple dans l'eau ou un solvant organique, ou bien sous forme de dispersion aqueuse ou organique.

Ils peuvent être utilisés dans les compositions cosmétiques ou pharmaceutiques à raison de 0,01 à 50% en poids de matière sèche, notamment de 0,1 à 30% en poids, voire de 0,3 à 10% en poids, encore mieux de 1 à 3% en poids, par rapport au poids total de la composition.

Les compositions cosmétiques ou pharmaceutiques selon l'invention comprennent, outre lesdits polymères, un milieu physiologiquement acceptable, notamment cosmétiquement ou pharmaceutiquement acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en C₂ et des aldéhydes en C₂-C₄ hydrophiles.
L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; leurs mélanges. Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.
Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.
On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther,ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.
La composition peut également comprendre d'autres matières colorantes choisies parmi les colorants hydrosolubles ou les colorants liposolubles bien connus de l'homme du métier.
Par pigments, il faut comprendre des particules de toute forme, blanches ou colorées, minérales ou organiques, insolubles dans le milieu physiologique, destinées à colorer la composition. Par charges, il faut comprendre des particules incolores ou blanches, minérales
ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01 % à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut également comprendre avantageusement au moins un agent tensioactif qui est généralement présent en une quantité comprise entre 0,01% et 50% en poids, de préférence entre 0,1% et 40% et encore plus préférentiellement entre 0,5% et 30%, par rapport au poids total de la composition.
Cet agent tensioactif peut être choisi parmi les agents tensioactifs anioniques, amphotères, non ioniques, cationiques ou leurs mélanges.
Les tensioactifs convenant à la mise en oeuvre de la présente invention sont notamment, seuls ou en mélange, :
- les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffinesulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.
   On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges.
- les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
- les tensioactifs amphotères parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.
- les tensioactifs cationiques parmi lesquels on peut citer, seuls ou mélanges,
   A) les sels d'ammonium quaternaires de la formule générale (XVI) suivante : 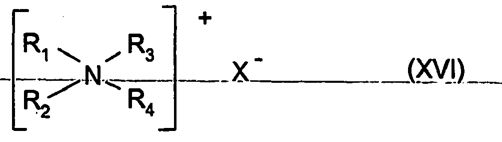 dans laquelle X est un anion choisi parmi les halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B)
      - les sels d'ammonium quaternaire de l'imidazolinium, comme par exemple celui de formule (XVII) suivante : 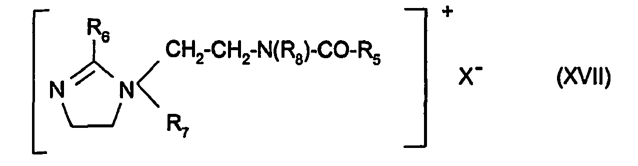 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C)
      - les sels de diammonium quaternaire de formule (XVIII) : 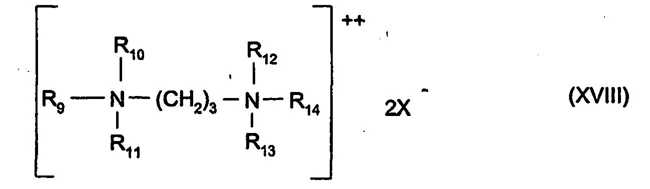 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D)
      - les sels d'ammonium quaternaire comprenant au moins une fonction ester de formule (XIX) suivante : 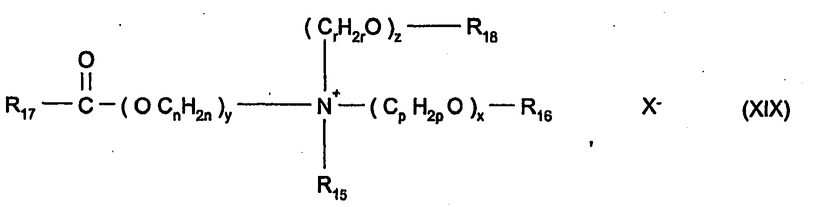 dans laquelle :
         - R15 est choisi parmi les radicaux alkyles en C₁-C₆ et les radicaux hydroxyalkyles ou dihydroxyalkyles en C1-C6 ;
         - R16 est choisi parmi :
            - le radical 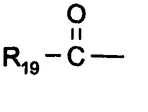
            - les radicaux R20 hydrocarbonés en C1-C22 linéaires ou ramifiés, saturés ou insaturés,
            - l'atome d'hydrogène,
         - R18 est choisi parmi :
            - le radical 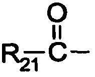
            - les radicaux R22 hydrocarbonés en C1-C6 linéaires ou ramifiés, saturés ou insaturés,
            - l'atome d'hydrogène,
         - R17, R19 et R21, identiques ou différents, sont choisis parmi les radicaux hydrocarbonés en C7-C22, linéaires ou ramifiés, saturés ou insaturés ;
         - n, p et r, identiques ou différents, sont des entiers valant de 2 à 6 ;
         - y est un entier valant de 1 à 10 ;
         - x et z, identiques ou différents, sont des entiers valant de 0 à 10 ;
         - X⁻ est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

La composition selon l'invention peut également comprendre des ingrédients couramment utilisés en cosmétique, tels que les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.
Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.
Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

L'invention a donc encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.
Ainsi, ce procédé selon l'invention permet le maintien de la coiffure, le traitement, le soin ou le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement au moins un tensioactif cationique, par exemple en une concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.
Les compositions selon l'invention peuvent également se présenter sous forme de lotions aqueuses ou hydroalcooliques pour le soin de la peau et/ou des cheveux.

L'invention a aussi pour objet un procédé cosmétique de maquillage ou de soin des matières kératiniques, notamment de la peau du corps ou du visage, des ongles, des cheveux et/ou des cils, comprenant l'application sur lesdites matières d'une composition cosmétique telle que définie précédemment.

L'invention est illustrée plus en détail dans les exemples suivants.

### Mesure de la Tg

Un film est réalisé à partir d'une solution aqueuse à 6% en poids de polymère et séché 48 heures dans une atmosphère contrôlée à 50% d'humidité relative et 25°C. Les films ainsi obtenus ont une épaisseur comprise entre 10 et 20 µm.
L'appareil de mesure est une DSC (TA instruments).
L'échantillon issu du film est déposé dans un creuset hermétique et est chauffé selon le protocole suivant :
- équilibre à température initiale Ti;
- chauffe 1 : augmentation de la température, à une vitesse de +10°C/min jusqu'à température finale :Tf (°C);
- isotherme durant 1 minute;
- diminution de la température à une vitesse de -10°C/min jusqu'à Ti (°C);
- chauffe2 : augmentation de la température à une vitesse de +10°C/min jusqu'à Tf (°C);
- isotherme durant 1 minute.
   avec Ti : Température initiale - 120°C
   avec Tf : Température finale + 120°C
Les Tg sont mesurées lors des étapes de chauffe 1 et 2.

### Mesure de la reprise en eau

On dépose environ 1 g de polymère sec dans une coupelle en aluminium de 4,5 cm de diamètre (0,01 m²). On laisse sécher 48 heures à l'étuve à 60°C, sous pression réduite. On retire les coupelles et on les pèse immédiatement (moins d'une minute après leur sortie de l'étuve). On obtient P1.
Les coupelles sont ensuite placées dans une boite à gant ayant l'humidité relative considérée (75% HR ou 85% HR) et y sont laissées 6 heures. Elles sont ensuite pesées à nouveau immédiatement après leur sortie de la boite à gant. On obtient P2.
La reprise en eau est calculée de la manière suivante : [(P2-P1) x 100] / P1

### Exemple 1

Dans un réacteur (quadricol) surmonté de deux ampoules d'addition, d'un réfrigérant et muni d'une agitation mécanique, on introduit 75 ml de méthyléthylcétone (MEC) , on porte à 80°C.
Parallèlement, on prépare une solution 1 comprenant les monomères : 50 g de méthacrylate de polyéthylèneglycol (MPEG 550), 50 g de diméthylaminopropylméthacrylamide (DMAPMA) et l'amorceur : 0,5 g de (Trigonox 21S).
On prépare également une solution 2 comprenant 75 ml de méthyléthylcétone et 0,5 g d'amorceur (Trigonox 21S).
La solution 1 est coulée goutte à goutte en 1 heure et la solution 2 en deux heures, dans le réacteur quadricol. Le mélange résultant est maintenu à 80°C ensuite pendant 5 heures. La solution jaune-orangée obtenue est refroidie. On obtient 95 g de polymère.
Le polymère présente une viscosité Brookfield à 15% dans la MEC, à 25°C, mesurée avec un mobile du type aiguille (spindle) numéro 1, à une vitesse de 0,1 tour/minute, de : 7,5 mPa.s.

On peut ensuite procéder à la neutralisation du polymère de la manière suivante : on ajoute 290 ml d'HCl 1N sous agitation au 95 g de polymère, et 200 ml d'eau distillée. Puis on procède à l'évaporation du solvant (MEC).
Le polymère neutralisé est soluble dans l'eau (au moins jusqu'à 50% en poids).
Sa Tg est de -60°C.
Le polymère neutralisé présente une reprise en eau à 85% HR de 51 %.

### Exemple 2

Dans un réacteur (quadricol) surmonté de deux ampoules d'addition, d'un réfrigérant et muni d'une agitation mécanique, on introduit 100 ml d'eau que l'on porte à 80°C.
Parallèlement, on prépare une solution 1 comprenant 50 g de monomère MPEG 550, 1 g d'amorceur (persulfate de potassium KPS) et 50 ml d'eau.
On prépare également une solution 2 comprenant 50 g de monomère DMAPMA neutralisé à 100% par le chlorhydrate de bétaïne et 50 g d'eau.
Les solutions 1 et 2 sont coulées en 1 heure dans le quadricol. Après une heure à 80°C, on y coule goutte à goutte en 15 minutes, un mélange de 1 g de KPS dans 50 ml d'eau.
Le mélange résultant est ensuite maintenu à 80°C pendant 3 heures. On obtient 90 g de polymère neutralisé par le chlorhydrate de bétaïne.

Le polymère présente une viscosité Brookfield à 15% dans l'eau, à 25°C, mesurée avec un mobile du type aiguille (spindle) numéro 1, à une vitesse de 6 tours/minute, de : 164 mPa.s.
Le polymère est soluble dans l'eau (au moins jusqu'à 50% en poids).
Sa Tg est de -60°C.
Le polymère neutralisé présente une reprise en eau à 85% HR de 90%.

### Exemples 3 à 14

On prépare, selon le procédé de l'exemple 1 (procédé solvant) ou de l'exemple 2 (procédé dans l'eau), les polymères selon l'invention suivants :

| Exemple | Monomères (% en poids) | | Procédé et neutralisation | solubilité |
|---|---|---|---|---|
| Exemple 3 | MPEG 550 | 10% | Procédé 1 | eau |
| | DMAPMA | 90% | HCl | |
| Exemple 4 | MPEG 1100 | 25% | Procédé 1 | eau |
| | DMAPMA | 75% | HCl | |
| Exemple 5 | MPEG 1100 | 50% | Procédé 1 | Eau |
| | DMAPMA | 50% | HCl | |
| Exemple 6 | MPEG 550 | 50% | Procédé 1 | Eau |
| | DMAPMA | 50% | HCl | |
| Exemple 7 | MPEG 550 | 50% | Procédé 2 | Eau |
| | SPE | 50% | Pas de neutr. | |
| Exemple 8 | MPEG 550 | 50% | Procédé 1 HCl | Eau |
| | MADAME | 50% | | |
| Exemple 9 | MPEG 550 | 50% | Procédé 1 | Eau |
| | Méthacrylate de morpholinoéthyle 50% | | HCl | |
| Exemple 10 | MPEG 2000 | 50% | Procédé 2 | Eau |
| | DMAPMA | 50% | Pas de neutr. | |
| Exemple 11 | MPEG 550 | 50% | Procédé 1 | Eau |
| | DMAPMA | 50% | Chlorhydrate | |
| | | | de bétaïne | |
| Exemple 12 | MPEG 550 | 40% | Procédé 1 | Eau |
| | DMAPMA | 35% | HCl | |
| | Acide acrylique | 15% | | |
| Exemple 13 | MPEG 550 | 50% | Procédé 1 | Dispersible dans l'eau |
| | DMAPMA | 50% | Acide béhénique à 20% | |
| | | | | |
| Exemple 14 | MPEG 550 | 50% | Procédé 1 | Dispersible dans l'eau |
| | DMAPMA | 50% | Acide oléïque | |

| | | | | |
|---|---|---|---|---|
| MPEG : méthacrylate de polyéthylèneglycol (avec PM = 550, 1100 ou 2000) DMAPMA : méthacrylamide de diméthylaminopropyle SPE : N,N-dimethyl-N-(2-methacryloyloxyethyl)-N-(3-sulfopropyl) ammonium bétaïne MADAME : méthacrylate de diméthylaminoéthyle | | | | |

### Exemple 15

On prépare une composition comprenant les constituants suivants (% en poids):
- 7,5% de lauryl éther sulfate
- 2,5% de tensioactif amphotère coco-bétaïne
- 5% de tensioactif coco-polyglucoside
- 1,5% de polymère de l'exemple 11
- qsp 100% eau

La composition de shampooing obtenue apporte un bon effet coiffant. La cosmétique sur cheveux humides est correcte. Les résultats coiffants obtenus après application sur têtes sont bons et les propriétés cosmétiques sur cheveux secs particulièrement bonnes. On constate que cette, composition apporte des propriétés cosmétiques équivalentes à celles apportées par un shampooing conditionneur usuel : cosmétique supérieure à l'état de la technique pour un coiffant équivalent; certains critères cosmétiques (lissage, souplesse et brillance) sont meilleurs avec la composition selon l'invention.

### Exemple 16

On prépare une composition comprenant les constituants suivants (% en poids):
- 7,5% de lauryl éther sulfate
- 2,5% de tensioactif amphotère coco-bétaïne
- 5% de tensioactif coco-polyglucoside
- 1,5% de polymère de l'exemple 13
- qsp 100% eau

On applique la composition résultante sur têtes en mono-application, et on évalue les critères suivants (panel de 6 personnes):
- sur cheveux humides : le lissage et le démêlage
- sur cheveux secs : ressort de la mèche enroulée et séchée (effet coiffant), démêlage, brillance.

On obtient les résultats suivants :
- sur cheveux humides, le démêlage et le lissage sont très bons.
- sur cheveux secs, l'effet coiffant est bon ; les propriétés cosmétiques sont bonnes, supérieures à l'état de la technique pour un effet coiffant équivalent ; certains critères cosmétiques (lissage, souplesse, brillance) sont meilleurs avec la composition selon l'invention.

Les résultats sont rassemblés dans le tableau ci-après :

| | Démêlage cheveu humide | Lissage cheveu humide | Démêlage cheveu sec | brillance | ressort |
|---|---|---|---|---|---|
| Composition de l'ex. 15 | +++ | +++ | +++ | +++ | ++ |
| Composition de l'ex. 16 | ++++ | ++++ | | ++++ | |
| Témoin (shampooing DOP camomille) | ++ | ++ | ++ | ++ | 0 |

### Exemple 17 : exemples comparatifs

Dans un premier temps, on prépare selon l'exemple 1, les polymères hors invention suivants :

| | | | |
|---|---|---|---|
| Comparatif 1 | MPEG 550 | 35% | Procédé 1 |
| (monomère additionnel hydrophobe) | DMAPMA | 50% | HCl |
| | acrylate d'éthylhexyle 15% | | |
| Comparatif 2 | MPEG 550 | 50% | Procédé 1 |
| (monomère additionnel hydrophobe) | DMAPMA | 15% | HCl |
| | Acrylate d'éthylhexyle 35% | | |
| Comparatif 3 | MPEG 550 | 50% | Procédé 1 |
| (polymère réticulé) | DMAPMA | 50% | HCl |
| | Diméthacrylate de butanediol 1% | | |
| Comparatif 4 | MPEG 550 | 94% | Procédé 1 |
| | DMAPMA | 6% | HCl |
| Comparatif 5 | MPEG 550 | 6% | Procédé 1 |
| | DMAPMA | 94% | Neutralisé HCl |
| Comparatif 6 | MPEG 550 | 50% | |
| | TMEACL* | 50% | |
| Comparatif 7 | Ethylvinyléther | 50% | Procédé 1 |
| (absence de MPEG) | DMAPMA | 50% | Neutralisé HCl |
| Comparatif 8 | MPEG 550 | 40% | Procédé 1 |
| | DMAPMA | 50% | Neutralisé HCl |
| | MAEE | 10% | |

| | | | |
|---|---|---|---|
| *TMEACL : acrylate de 2-(diméthylamino)éthyle, quaternisé par du chlorure de méthyle * MAEE : méthacrylate d'éthoxyéthyle | | | |

On prépare une composition comprenant les constituants suivants (% en poids):
- 7,5% de lauryl éther sulfate
- 2,5% de tensioactif amphotère coco-bétaïne (Dehyton AB30 de Cognis)
- 5% de tensioactif coco-polyglucoside (Plantacare 818 UP de Cognis)
- 3% de polymère (ou mélange de polymères)
- qsp 100% eau

On applique la composition résultante sur têtes en mono-application, et on évalue les critères suivants (panel de 6 personnes):
- sur cheveux humides : le lissage et le démêlage
- sur cheveux secs : ressort de la mèche enroulée et séchée (effet coiffant)

On compare ces compositions hors invention avec une composition selon l'invention comprenant le polymère de l'exemple 11 (composition C1).

Les résultats sont rassemblés dans les tableaux ci-après :

| Polymère contenu dans la composition | Observations |
|---|---|
| Polymère comparatif 4 | - sur cheveux humides : démêlage et lissage inférieurs à la composition C1 - sur cheveux secs : ressort inférieur à la composition C1 - non coiffant |
| Polymère comparatif 5 | - sur cheveux humides : démêlage et lissage inférieurs à la composition C1 - sur cheveux secs : ressort inférieur à la composition C1 - non coiffant |
| Polymère comparatif 6 | - sur cheveux humides : démêlage et lissage inférieurs à la composition C1; mèche moins glissante qu'avec C1 - sur cheveux secs : ressort inférieur à la composition C1; boucle plus détendue, moins tonique, qu'avec C1; toucher plus crissant qu'avec C1 |
| Polymère comparatif 7 | - sur cheveux humides : démêlage et lissage inférieurs à la composition C1; mèche plus crissante qu'avec C1 - sur cheveux secs : ressort inférieur à la composition C1; boucle plus détendue, moins tonique, qu'avec C1 |
| Polymère comparatif 8 | - sur cheveux humides : démêlage et lissage inférieurs à la composition C1 - sur cheveux secs : ressort proche de la composition C1 |

| Polymère présent dans la composition | Démêlage cheveu humide | toucher | Ressort | coiffant |
|---|---|---|---|---|
| Exemple 11 | +++ | + | ++ | ++ |
| Mélange 50/50 DMAPMA/polyéthylèneglycol | +++ problème de mousse | | + | 0 |
| Polymère comparatif 1 | ++ | - | + | 0 |
| Polymère comparatif 2 | ++ | - | +++ | 0 |
| Polymère comparatif 3 | + | - | +++ | 0 |

### Exemple 18

On prépare une composition comprenant les constituants suivants (% en poids):
- 6% de polymère de l'exemple 1
- 0,001 % de conservateur
- qsp 100% eau

La composition est introduite dans un flacon-pompe; elle est appliquée sur cheveux secs et coiffés et l'on évalue les critère suivants: ressort de la mèche enroulée et séchée (effet coiffant). On constate que l'effet coiffant est très bon : la mèche traitée est facilement disciplinée; les cheveux présentent un meilleur volume par rapport à l'état de la technique.

### Exemple 19

On prépare un shampoing comprenant les constituants suivants (% en poids):
- 7,5% de lauryl éther sulfate
- 2,5% de tensioactif amphotère coco-bétaïne (Dehyton AB30 de Cognis)
- 5% de tensioactif coco-polyglucoside (Plantacare 818 UP de Cognis)
- qs conservateur
- 1,5% de matière active de polymère
- qsp 100% eau

On applique la composition résultante sur têtes en mono-application, et on évalue les critères suivants (panel de 4 personnes):
- sur cheveux humides : le lissage, la légèreté et la facilité de démêlage
- sur cheveux secs : ressort de la mèche enroulée et séchée (effet coiffant)

On obtient les résultats suivants :

| | |
|---|---|
| Polymère | MPEG550/DMAPMA 50/50 neutralisé chlorhydrate de bétaïne |

- Sur cheveux humides : la composition apporte du lissage, de la facilité de démêlage et de la légèreté;
- Sur cheveux séchés : la composition donne de la masse, de la souplesse, du volume et du lissage à la chevelure.

## Revendications

1. Copolymère éthylénique constitué essentiellement, en % en poids par rapport au poids total du polymère :
- a) de 10% inclus à 60% exclus en poids d'au moins un monomère de formule (I), et/ou ses sels : 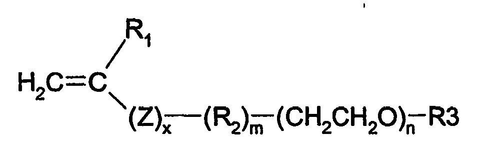 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO- , - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P; et
- b) de 40% exclus à 90% inclus en poids d'au moins un monomère "essentiellement cationique", et/ou ses sels, choisi parmi :
- (i) un ou plusieurs monomères cationiques de formule (IIa),
- (ii) un ou plusieurs monomères amphotères de formules (IIc) et (IId), et
- (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId). 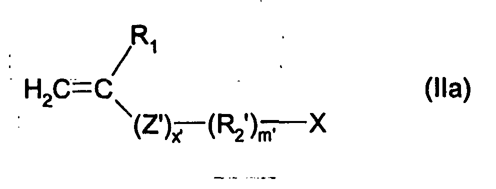 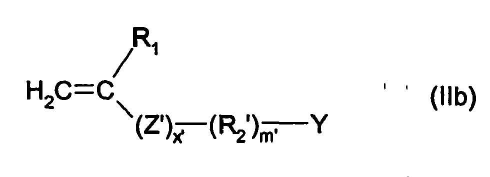 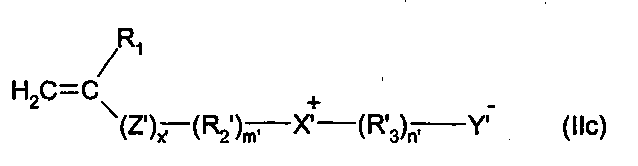 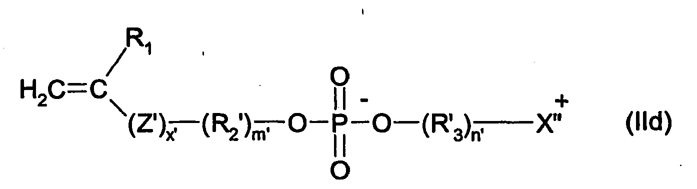
dans lesquelles :
- R1 est un atome d'hydrogène ou un radical hydrocarboné, linéaire ou ramifié, de type CₚH₂ₚ₊₁, avec p étant un entier compris entre 1 et 12, inclus; de préférence l'hydrogène ou un radical méthyle, éthyle, propyle, butyle.
- Z' est un groupement divalent choisi parmi -COO-, -CONH-, -CONCH₃-, -OCO- ou -O-, -SO₂- -CO-O-CO- ou -CO-CH₂-CO-; de préférence, COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m' est 0 ou 1 ;
- X (dans la formule IIa) est
(a) un groupe guanidino, amidino, ou bien
(b) un groupe de formule -N(R₆)(R₇) ou -P(R₆)(R₇) ou -P⁺R₆R₇R₈, avec R6, R7 et R8 représentant, indépendamment l'un de l'autre, soit (i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; soit (iii) R6 et R7 peuvent former avec l'atome d'azote ou de phosphore un premier cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi -O; S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ; ou bien
(c) X représente un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.
- Y est un groupement choisi parmi -COOH, -SO₃H, -OSO₃H, -PO₃H₂ et -OPO₃H₂.
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.
- Y'⁻ est un groupement choisi parmi -COO⁻, -SO₃⁻' -OSO₃⁻, -PO₃²⁻ et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
- n' est compris entre 1 et 100, de préférence 1 et 5 inclus;
- X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N;
ainsi que les sels dudit copolymère:

2. Polymère selon la revendication 1 dans lequel, dans la formule (1), R1 représente l'hydrogène ou un radical méthyle, éthyle, propyle, butyle.

3. Polymère selon l'une des revendications précédentes, dans lequel, dans la formule (I), Z représente COO ou CONH.

4. Polymère selon l'une des revendications précédentes, dans lequel, dans la formule (I), le radical R2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule: 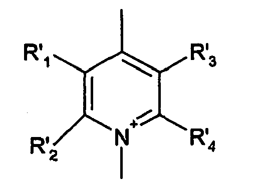 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-CHOH-, -CH₂-CH₂-CHOH-, -CH₂-CH₂-CH(NH₂)-, - CH₂-CH(NH₂)-, -CH₂-CH₂-CH(NHR'), -CH₂-CH(NHR')-, -CH₂-CH₂-CH(NR'R")-, - CH₂-CH(NR'R")-, -CH₂-CH₂-CH₂-NR'-, -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH- ou -CH₂-CH₂-NH-CO-NH- ; -CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

5. Polymère selon l'une des revendications précédentes, dans lequel, dans la formule (I), n est compris entre 5 et 200 inclus, et encore mieux entre 7 et 100 inclus, voire entre 9 et 50 inclus.

6. Polymère selon l'une des revendications précédentes, dans lequel, dans la formule (I), R3 est un atome d'hydrogène; un radical succinimido, maléimido, mésityle, tosyle, triéthoxysilane, phtalimide ou -CH₂-CH₂CN; ou encore un radical benzyle ou phényle éventuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P ;
lesdits radicaux benzyle, phényle ou alkyle pouvant comprendre en outre une fonction choisie parmi les fonctions suivantes: Succinimido ; Glutarate-succinimido ; Glutarate ; maléimido ; Mésityle ; Benzoate ; Tosyle ; Triéthoxysilane ; Phtalimide ; Thioester ; Benzotriazole carbonate ; Butyraldéhyde ; Acétaldéhyde diéthylacétal ; Biotine ; Phospholipide ; Succinate ; N-hydroxysuccinimide ; - SO₃H, -COOH, -PO₄, -NR5R6 ou -N⁺ R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore des groupements protecteurs tels que le t-butyloxycarbonyle ou le 9-fluorenylmethoxycarbonyle.

7. Polymère selon l'une des revendications précédentes, dans lequel le monomère de formule (I) est choisi parmi, seul ou en mélange :
- le (méth)acrylate de poly(éthylène glycol) ;
- le (méth)acrylate de méthyl-poly(éthylène glycol) ;
- les (méth)acrylate d'alkyl-poly(éthylène glycol) ;
- les (méth)acrylates de phényl-poly(éthylène glycol);
- le monomère suivant : 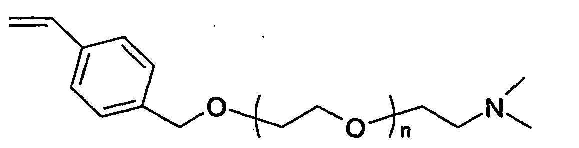 dans lequel n est de préférence compris entre 3 et 100 inclus, notamment 5 à 50 inclus, voire 7 à 30 inclus.

8. Polymère selon l'une des revendications précédentes, dans lequel le monomère de formule **(I)** a un poids moléculaire compris entre 350 et 13 000 g/mol., notamment entre 500 et 8000 g/mol.

9. Polymère selon l'une des revendications précédentes, dans lequel le monomère de formule (I), seul où en mélange, est choisi parmi les (méth)acrylates de poly(éthylène glycol) et les (méth)acrylates de méthyl-poly(éthylène glycol), de préférence ceux ayant un poids moléculaire compris entre 350 et 13 000 g/mol., notamment entre 500 et 8000 g/mol.

10. Polymère selon l'une des revendications précédentes, dans lequel le monomère de formule (I), seul ou en mélange, est choisi parmi les (méth)acrylates de poly(éthylène glycol), en particulier ceux ayant un poids moléculaire entre 350 et 13 000 g/mol., notamment entre 500 et 8000 g/mol.

11. Polymère selon l'une des revendications précédentes, dans lequel le monomère de formule (I), seul ou en mélange, est présent à raison de 20% inclus à 55% inclus en poids, par rapport au poids du polymère final, de préférence de 30% inclus à 50% inclus en poids.

12. Polymère selon l'une, des revendications précédentes, dans lequel dans les formules (IIa), (IIb), (IIc) et/ou (IId), le radical R'2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;

13. Polymère selon l'une des revendications précédentes, dans lequel dans la formule (IIa), les radicaux R6 et R7 présents dans X sont choisis parmi l'hydrogène, un groupement méthyle, éthyle, propyle, isopropyle, n-butyle, t-butyle, isobutyle, octyle, lauryle, stéaryle.

14. Polymère selon l'une des revendications précédentes, dans lequel dans la formule (IIa), X est un radical choisi parmi les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

15. Polymère selon l'une des revendications précédentes, dans lequel le monomère de formule (IIa) est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle, et les monomères ci-après :

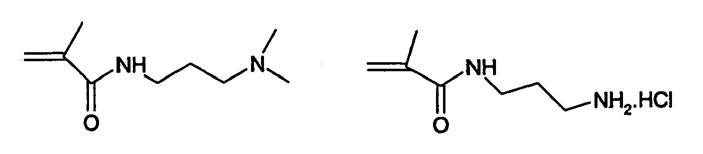

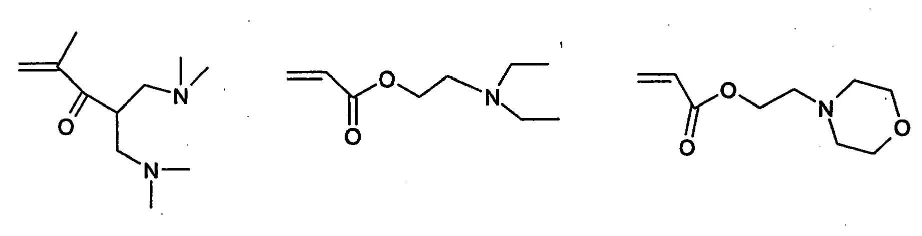

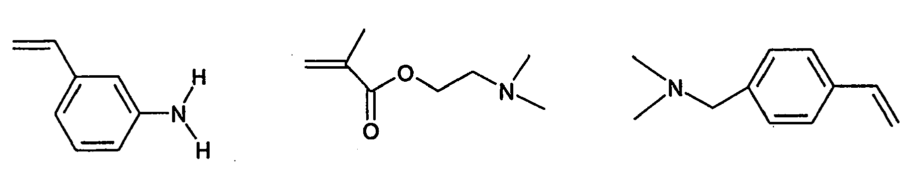

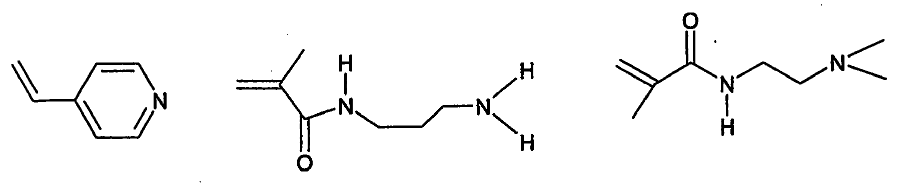

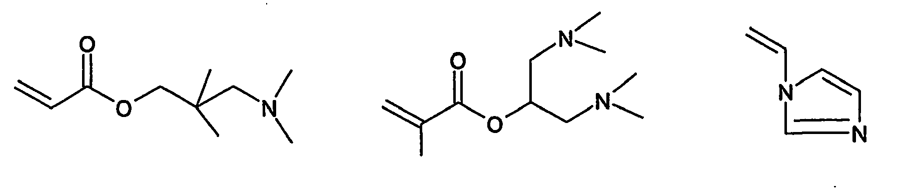

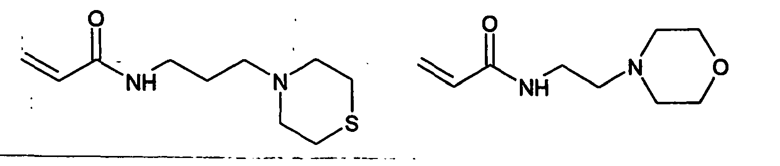

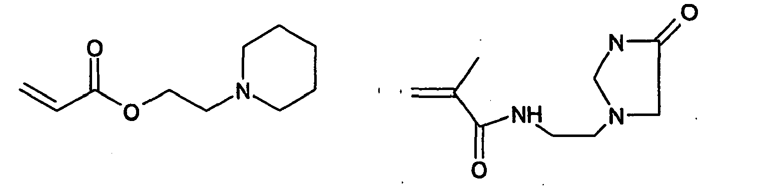

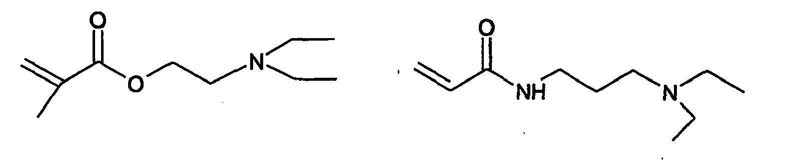

16. Polymère selon l'une des revendications précédentes, dans lequel les monomères de formule (IIa) sont choisis parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle.

17. Polymère selon l'une des revendications précédentes, dans lequel les monomères anioniques sont choisis parmi l'anhydride maléique, l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.

18. Polymère selon l'une des revendications précédentes, dans lequel dans la formule (IIc), le radical X'⁺ est choisi parmi les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

19. Polymère selon l'une des revendications précédentes, dans lequel dans les formules (IIc) et/ou (IId), le radical R'3 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi O, N, S, F, Si et P; ou bien un radical benzylène -C₆H₄-CH₂- éventuellement substitué, par un radical alkyle en C1-C12 comprenant éventuellement 1 à 5 hétéroatomes choisis par parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -[CH₂-CH₂-O]r,- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;

20. Polymère selon l'une des revendications précédentes, dans lequel dans la formule (IId), X"⁺ est choisi parmi les radicaux triméthylammonium; triéthylammonium; N,N-diméthyl,N-octylammonium; N,N-diméthyl,N-laurylammonium.

21. Polymère selon l'une des revendications précédentes, dans lequel le monomère de formules (IIc) ou (IId) sont choisis parmi la N,N-dimethyl-N-(2-methacryloyloxyethyl)-N-(3-sulfopropyl) ammonium bétaïne, la N,N-dimethyl-N-(3-methacrylamidopropyl)-N-(3-sulfopropyl) ammonium bétaïne, la 1-(3-sulfopropyl)-2-vinylpyridinium bétaïne et la 2-méthacryloyloxyéthylphosphorylcholine.

22. Polymère selon l'une des revendications précédentes, dans lequel, lorsque le monomère 'essentiellement cationique' est choisi parmi les mélanges de monomères cationiques et/ou amphotères avec des monomères anioniques, lesdits monomères anioniques sont de préférence présents à raison de 5 à 40% en poids, par rapport au poids du mélange "cationiques et/ou amphotères + anioniques", notamment de 10 à 30% en poids, de préférence de 15 à 25% en poids.

23. Polymère selon l'une des revendications précédentes, dans lequel le monomère 'essentiellement cationique' est présent à raison de 45% inclus à 80% inclus en poids par rapport au poids du polymère final, de préférence de 50% inclus à 70% inclus en poids.

24. Polymère selon l'une des revendications précédentes, dans lequel au moins un des monomères, et/ou le polymère est neutralisé par un agent neutralisant choisi parmi les bases minérales, les bases organiques, les acides minéraux et/ou les acides organiques.

25. Polymère selon l'une des revendications précédentes, dans lequel au moins un des monomères, et/ou le polymère, est neutralisé par un agent neutralisant de type acide minéral ou organique, ayant un log P inférieur ou égal à 2, par exemple compris entre -8 et 2, de préférence compris entre -6 et 1, notamment compris entre -6 et 0 ; ou bien ayant un log P supérieur à 2, de préférence supérieur ou égal à 2,5, notamment supérieur à 3, et en particulier compris entre 3 et 15, voire entre 3,5 et 10.

26. Polymère selon l'une des revendications précédentes, dans lequel au moins un des monomères, et/ou le polymère, est neutralisé par un agent neutralisant de type acide minéral ou organique, ayant un log P inférieur ou égal à 2, par exemple compris entre -8 et 2, de préférence compris entre -6 et 1, notamment compris entre -6 et 0.

27. Polymère selon l'une des revendications précédentes, dans lequel au moins un des monomères, et/ou le polymère, est neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

28. Polymère selon l'une des revendications précédentes, dans lequel :
- le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) et/ou les (méth)acrylates de méthyl-poly(éthylène glycol), de préférence ceux ayant un poids moléculaire compris entre 350 et 13 000 g/mol., notamment entre 500 et 8000 g/mol.; et/ou
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisi parmi, seul ou en mélange, les monomères de formule (IIb).

29. Polymère selon l'une des revendications précédentes, dans lequel :
- le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisis parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle.

30. Polymère selon l'une des revendications précédentes, dans lequel :
- le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle; et
- le polymère est neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

31. Polymère selon l'une des revendications précédentes, dans lequel:
- le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40% exclus à 90% inclus en poids par rapport au poids du polymère final, notamment de 45% inclus à 80% inclus en poids, de préférence de 50% inclus à 70% inclus en poids et est choisi parmi, seul ou en mélange, le (méth)acrylamide de diméthylaminopropyle, et
- le polymère est neutralisé par un agent neutralisant choisi parmi l'acide béhénique et/ou le chlorhydrate de bétaïne.

32. Polymère selon l'une des revendications précédentes, présentant une masse moléculaire moyenne en poids (Mw) comprise entre 500 et 5 000 000, notamment comprise entre 1 000 et 3 000 000 et plus préférentiellement comprise entre 2 000 et 2 000 000, voire 4 000 et 500 000, entre autre 7 000 et 400 000, encore mieux 20 000 et 350 000, et encore 150 000 et 300 000.

33. Polymère selon l'une des revendications précédentes, **caractérisée par le fait qu'**il est véhiculable en milieu aqueux, de préférence hydrosoluble ou hydrodispersible.

34. Polymère selon l'une des revendications précédentes, **caractérisée par le fait qu'**il présente une viscosité dans l'eau comprise entre 1 et 1000 mPa.s, de préférence entre 1,5 et 750 mPa.s, et encore mieux entre 2 et 500 mPa.s, mesurée à 25°C, à l'aide d'un viscosimètre BROOKFIELD, pour une solution à 15% en poids de polymère dans l'eau ou la méthyléthylcétone, à 25°C, avec un mobile de type aiguille (spindle) no 1 ; pour un temps de mesure de 5 minutes, à une vitesse comprise entre 0,1 et 6 tours/minute.

35. Polymère selon l'une des revendications précédentes, **caractérisé par le fait qu'**il présente une température de transition vitreuse (Tg) comprise entre -150°C et 20°C, notamment -120°C et 10°C, mieux entre -100°C et 0°C.

36. Polymère selon l'une des revendications précédentes, **caractérisé par le fait qu'**il présente une reprise en eau comprise entre 3% et 150% en poids, de préférence entre 4% et 100% en poids, notamment entre 5% et 50% en poids, à 75% d'humidité relative (75%HR) ; et/ou qu'il présente une reprise en eau comprise entre 3% et 200% en poids, de préférence entre 2,5% et 150% en poids, notamment entre 3% et 100% en poids, à 85% d'humidité relative (85%HR).

37. Composition cosmétique ou pharmaceutique comprenant dans un milieu physiologiquement acceptable, notamment cosmétiquement ou pharmaceutiquement acceptable, au moins un polymère tel que défini à l'une des revendications 1 à 36.

38. Composition selon la revendication 37, dans laquelle le polymère est présent à raison de 0,01 à 50% en poids de matière sèche, notamment de 0,1 à 30% en poids, voire de 0,3 à 10% en poids, encore mieux de 1 à 3% en poids, par rapport au poids total de la composition.

39. Composition selon l'une des revendications 37 à 38, dans laquelle le milieu physiologiquement acceptable comprend au moins un constituant choisi parmi l'eau ; les solvants organiques hydrophiles comme les alcools, notamment les monoalcools, linéaires ou ramifiés en C1-C6 et les polyols et les éthers de glycols notamment en C₂ et des aldéhydes en C₂-C₄ hydrophiles ; les cires, les corps gras pâteux, les gommes et leurs mélanges, d'origine animale, végétale, minérale ou synthétique ; des solvants organiques lipophiles ; des huiles d'origine animale, végétale, minérale ou synthétique ; les esters et les éthers de synthèse ; les esters du pentaérythritol; des alcools gras ayant de 12 à 26 atomes de carbone ; les huiles fluorées partiellement hydrocarbonées et/ou siliconées; les huiles siliconées volatiles ou non, linéaires ou cycliques, liquides ou pâteux à température ambiante; des pigments, des nacres, des charges ; les colorants hydrosolubles, les colorants liposolubles les polymères ; les agents auxiliaires de filmification ; les tensioactifs ; les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides ; leurs mélanges.

40. Composition selon l'une des revendications 37 à 39, se présentant sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

41. Composition selon l'une des revendications 37 à 40, se présentant sous la forme d'un produit de soin et/ou de maquillage de la peau du corps ou du visage, des lèvres et des cheveux, d'un produit solaire ou autobronzant, d'un produit capillaire.

42. Composition selon l'une des revendications 37 à 41, se présentant sous la forme d'une composition capillaire, notamment pour le maintien de la coiffure ou la mise en forme des cheveux, par exemple sous forme de shampooings, de gels, de lotions de mise en plis, de lotions pour le brushing, de compositions de fixation et de coiffage telles que les laques ou spray ; d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

43. Composition selon l'une des revendications 37 à 42, comprenant (% en poids):
- 7,5% de lauryl éther sulfate
- 2,5% de tensioactif amphotère coco-bétaïne
- 5% de tensioactif coco-polyglucoside
- 1,5% de polymère selon l'invention, et
- qsp 100% eau;
ledit polymère comprenant 50% en poids de méthacrylate de poly(éthylène glycol) de poids moléculaire de 550 g/mol. et 50% en poids de méthacrylamide de diméthylaminopropyle, et étant neutralisé par le chlorhydrate de bétaïne.

44. Composition selon la revendication 43, se présentant sous la forme d'un shampoing.

45. Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 37 à 44.

## Claims

1. Ethylenic copolymer essentially constituted of, as a weight percentage relative to the total weight of the polymer:
- a) from 10% inclusive to 60% exclusive by weight of at least one monomer of formula (I) and/or salts thereof: 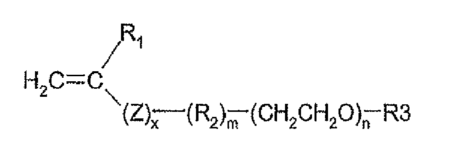 in which:
- R1 is a hydrogen atom or a linear or branched hydrocarbon-based radical, of the type CpH₂ₚ₊₁, with p being an integer between 1 and 12 inclusive;
- Z is a divalent group chosen from -COO-, -CONCH-, -CONCH₃-, -OCO-, -O-, -SO₂-, -CO-O-CO- and -CO-CH₂-CO-; - x is 0 or 1;
- R2 is a saturated or unsaturated, optionally aromatic, linear, branched or cyclic carbon-based divalent radical of 1 to 30 carbon atoms, possibly comprising 1 to 18 heteroatoms chosen from 0, N, S, F, Si and P;
- m is 0 or 1;
- n is an integer between 3 and 300 inclusive;
- R3 is a hydrogen atom or a saturated or unsaturated, optionally aromatic, linear, branched or cyclic carbon-based radical of 1 to 30 carbon atoms, possibly comprising 1 to 20 heteroatoms chosen from 0, N, S, F, Si and P; and
- b) from 40% exclusive to 90% inclusive by weight of at least one "essentially cationic" monomer, and/or salts thereof, chosen from:
- (i) one or more cationic monomers of formula (IIa),
- (ii) one or more amphoteric monomers of formulae (IIc) and (IId), and
- (iii) a mixture of one or more cationic monomers of formula (IIa) with one or more anionic monomers chosen from maleic anhydride and/or those of formula (IIb); and/or with one or more amphoteric monomers chosen from those of formulae (IIc) and (IId). 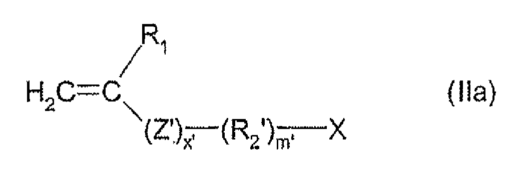 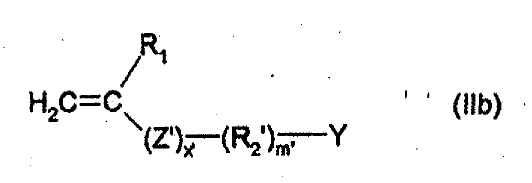 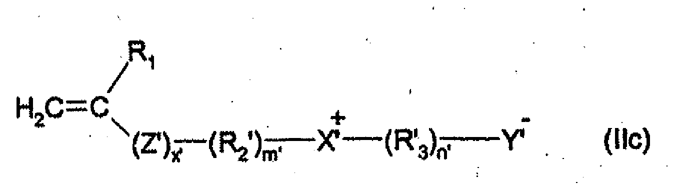 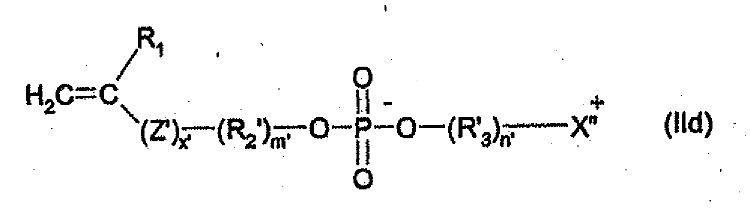
in which:
- R1 is a hydrogen atom or a linear or branched hydrocarbon-based radical of the type CₚH_{2p+1,} with p being an integer between 1 and 12 inclusive; preferably hydrogen or a methyl, ethyl, propyl or butyl radical;
- Z' is a divalent group chosen from -COO-, -CONH-, -CONCH₃-, -OCO- or -0-, -SO₂- -CO-O-CO- or -CO-CH₂-CO-; preferably COO and CONH;
- x' is 0 or 1, preferably 1;
- R'2 is a saturated or unsaturated, optionally aromatic, linear, branched or cyclic divalent carbon-based radical of 1 to 30 carbon atoms, possibly comprising 1 to 18 heteroatoms chosen from 0, N, S, F, Si and P;
- m' is 0 or 1;
- X (in formula IIa) is
(a) a guanidino or amidino group, or
(b) a group of formula -N (R₆) (R₇) or -P (R₆) (R₇) or -P⁺R₆R₇R₈, with R6, R7 and R8 representing, independently of each other, either (i) a hydrogen atom, or (ii) a linear, branched or cyclic, saturated or unsaturated, optionally aromatic alkyl group containing from 1 to 18 carbon atoms, possibly comprising 1 to 10 heteroatoms chosen from O, N, S, F, Si and P; or (iii) R6 and R7 may form with the nitrogen or phosphorus atom a first saturated or unsaturated, optionally aromatic ring comprising in total 5, 6, 7 or 8 atoms, and especially 4, 5 or 6 carbon atoms and/or 2 to 4 heteroatoms chosen from 0, S and N; said first ring possibly being fused with one or more other saturated or unsaturated, optionally aromatic rings each comprising 5, 6 or 7 atoms, and especially 4, 5, 6 or 7 carbon atoms and/or 2 to 4 heteroatoms chosen from 0, S and N; or
(c) X represents a group -R'6-N-R'7- in which R' 6 and R'7 form with the nitrogen atom a saturated or unsaturated, optionally aromatic ring comprising in total 5, 6, 7 or 8 atoms, and especially 4, 5 or 6 carbon atoms and/or 2 to 4 heteroatoms chosen from 0, S and N; said ring possibly being fused with one or more other saturated or unsaturated, optionally aromatic rings, each comprising 5, 6 or 7 atoms, and especially 4, 5, 6, 7 or 8 carbon atoms and/or 2 to 4 heteroatoms chosen from 0, S and N;
- Y is a group chosen from -COOH, -SO₃H, -OSO₃H, -PO₃H₂ and -OPO₃H₂;
- X'⁺ is a divalent group of formula -N⁺ (R₆) (R₇) - with R6 and R7 representing, independently of each other, either (i) a hydrogen atom, or (ii) a linear, branched or cyclic, optionally aromatic alkyl group containing from 1 to 25 carbon atoms, possibly comprising 1 to 20 heteroatoms chosen from O, N, S and P; or (iii) R6 and R7 may form with the nitrogen atom a first saturated or unsaturated, optionally aromatic ring comprising in total 5, 6, 7 or 8 atoms, and especially 4, 5, 6 or 7 carbon atoms and/or 2 to 3 heteroatoms chosen from O, S and N; said first ring possibly being fused with one or more other saturated or unsaturated, optionally aromatic rings, each comprising 5, 6, 7 or 8 atoms, and especially 4, 5, 6 or 7 carbon atoms and/or 2 to 3 heteroatoms chosen from O, S and N;
- Y'⁻ is a group chosen from -COO⁻, -SO₃⁻, -OSO₃⁻, -PO₃²⁻ and -OPO₃²⁻;
- R'3 is a saturated or unsaturated, optionally aromatic, linear, branched or cyclic divalent carbon-based radical of 1 to 30 carbon atoms, possibly comprising 1 to 18 heteroatoms chosen from 0, N, S, F, Si and P;
- n' is between 1 and 100 and preferably between 1 and 5 inclusive;
- X"⁺ is a group of formula -N⁺R₆R₇R₈ with R6, R7 and R8 representing, independently of each other, either (i) a hydrogen atom, or (ii) a linear, branched or cyclic, optionally aromatic alkyl group containing from 1 to 18 carbon atoms, possibly comprising 1 to 5 heteroatoms chosen from O, N, S and P; or (iii) R6 and R7 may form with the nitrogen atom a first saturated or unsaturated, optionally aromatic ring comprising in total 5, 6 or 7 atoms, and especially 4, 5 or 6 carbon atoms and/or 2 to 3 heteroatoms chosen from O, S and N; said first ring possibly being fused with one or more other saturated or unsaturated, optionally aromatic rings, each comprising 5, 6 or 7 atoms, and especially 4, 5, 6 or 7 carbon atoms and/or 2 to 3 heteroatoms chosen from O, S and N;
and the salts of said copolymer.

2. Polymer according to Claim 1, in which, in formula (I), R1 represents hydrogen or a methyl, ethyl, propyl or butyl radical.

3. Polymer according to either of the preceding claims, in which, in formula (I) , Z represents COO or CONH.

4. Polymer according to one of the preceding claims, in which, in formula (I), the radical R2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 25 heteroatoms chosen from 0, N, S, F, Si and P; or alternatively a benzylene radical -C₆H₄-CH₂-, optionally substituted with a C1-C12 alkyl radical optionally comprising 1 to 8 heteroatoms chosen from O, N, S, F, Si and P;
- a pyridinium radical of formula: 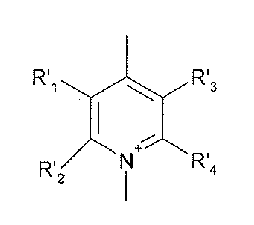 with R'1 to R' 4, which may be identical or different, chosen from H and a C1-C12 alkyl radical optionally comprising 1 to 8 heteroatoms chosen from 0, N, S, F, Si and P; R'1 to R'4 may especially be methyl and/or ethyl;
- a radical of formula -CH₂-CHOH-, -CH₂-CH₂-CHOH-, -CH2-CH₂-CH (NH₂) -CH₂-CH(NH₂)-, -CH₂-CH₂-CH (NHR') -, -CH₂-CH (NHR') -, -CH₂-CH₂-CH (NR' R") -, -CH₂-CH(NR' R")-, -CH₂-CH₂-CH₂-NR'-, -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH- or -CH₂-CH₂-NH-CO-NH-; -CH₂-CH₂-CH₂-O-; -CH₂-CH₂-CHR'-O- with R' and R" representing a linear or branched C1-C22 alkyl optionally comprising 1 to 12 heteroatoms chosen from 0, N, S, F, Si and P;
- or a mixture of these radicals.

5. Polymer according to one of the preceding claims, in which, in formula (I), n is between 5 and 200 inclusive and better still between 7 and 100 inclusive, or even between 9 and 50 inclusive.

6. Polymer according to one of the preceding claims, in which, in formula (I) , R3 is a hydrogen atom; a succinimido, maleimido, mesityl, tosyl, triethoxysilane, phthalimide or -CH₂-CH₂CN radical; or alternatively a benzyl or phenyl radical optionally substituted with a C1-C12 alkyl radical optionally comprising 1 to 8 heteroatoms chosen from 0, N, S, F, Si and P; a C1-C30 and especially C1-C22 or even C2-C16 alkyl radical, optionally comprising 1 to 18 heteroatoms chosen from 0, N, S, F, Si and P;
said benzyl, phenyl or alkyl radicals also possibly comprising a function chosen from the following functions: succinimido; glutarate-succinimido; glutarate; maleimido; mesityl; benzoate; tosyl; triethoxysilane; phthalimide; thioester; benzotriazole carbonate; butyraldehyde; acetaldehyde diethyl acetal; biotin; phospholipid; succinate; N-hydroxysuccinimide; -SO₃H, -COOH, -PO₄, -NR5R6 or -N⁺R5R6R7, with R5, R6 and R7, independently of each other, chosen from H and linear, branched or cyclic C1-C18 alkyls, especially methyl, optionally comprising one or more heteroatoms or alternatively protecting groups such as t-butyloxycarbonyl or 9-fluorenylmethoxycarbonyl.

7. Polymer according to one of the preceding claims, in which the monomer of formula (I) is chosen, alone or as a mixture, from:
- poly(ethylene glycol) (meth)acrylate;
- methylpoly(ethylene glycol) (meth)acrylate;
- alkylpoly(ethylene glycol) (meth)acrylates;
- phenylpoly(ethylene glycol) (meth)acrylates;
- the following monomer: 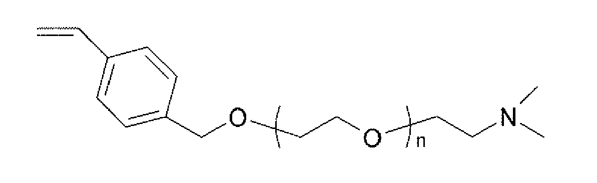 in which n is preferably between 3 and 100 inclusive and especially 5 to 50 inclusive, or even 7 to 30 inclusive.

8. Polymer according to one of the preceding claims, in which the monomer of formula (I) has a molecular weight of between 350 and 13 000 g/mol and especially between 500 and 8000 g/mol.

9. Polymer according to one of the preceding claims, in which the monomer of formula (I), alone or as a mixture, is chosen from poly(ethylene glycol) (meth)acrylates and methylpoly(ethylene glycol) (meth)acrylates, preferably those with a molecular weight of between 350 and 13 000 g/mol and especially between 500 and 8000 g/mol.

10. Polymer according to one of the preceding claims, in which the monomer of formula (I), alone or as a mixture, is chosen from poly(ethylene glycol) (meth)acrylates and in particular those with a molecular weight of between 350 and 13 000 g/mol and especially between 500 and 8000 g/mol.

11. Polymer according to one of the preceding claims, in which the monomer of formula (I), alone or as a mixture, is present in a proportion of from 20% inclusive to 55% inclusive by weight and preferably from 30% inclusive to 50% inclusive by weight relative to the weight of the final polymer.

12. Polymer according to one of the preceding claims, in which, in formulae (IIa), (IIb), (IIc) and/or (IId), the radical R'2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 5 heteroatoms chosen from N, 0, S, F, Si and/or P; or alternatively a benzylene radical -C₆H₄-CH₂-, optionally substituted with a C1-C12 alkyl radical optionally comprising 1 to 5 heteroatoms chosen from 0, N, S, F, Si and P;
- a radical of formula -CH₂-O-CO-O-, CH2-CH₂-O-CO-O-, -CH₂-CO-O-, -CH₂-CH₂-CO-O-, - [ (CH₂) ₅-CO-O] ₙ-, -CH₂-CH (CH₃) -O-, - (CH₂) ₂-O-, -CH₂-O-CO-NH-, -CH₂-CH₂-O-CO-NH-; -CH₂-NH-CO-NH- or -CH₂-CH₂-NH-CO-NH-, -CH₂-CHOH-, -CH₂-CH₂-CHOH-, -CH₂-CH₂-CH (NH₂) -, -CH₂-CH (NH₂) -, -CH₂-CH₂-CH (NHR') -, -CH₂-CH (NHR') -, -CH₂ -CH₂-CH (NR' R") -, -CH₂-CH (NR' R") -, -CH₂-CH₂-CH₂-NR' -, -CH₂-CH₂-CH₂-O-; -CH₂-CH₂-CHR'-O- with R' and R" representing a linear or branched C1-C22 alkyl optionally comprising 1 to 12 heteroatoms chosen from 0, N, S, F, Si and P;
- or a mixture of these radicals.

13. Polymer according to one of the preceding claims, in which, in formula (IIa), the radicals R6 and R7 present in X are chosen from hydrogen and a methyl, ethyl, propyl, isopropyl, n-butyl, t-butyl, isobutyl, octyl, lauryl or stearyl group.

14. Polymer according to one of the preceding claims, in which, in formula (IIa), X is a radical chosen from radicals of pyridine, indolyl, isoindolinyl, imidazolyl; imidazolinyl, piperidyl, pyrazolinyl, pyrazolyl, quinoline, pyrazolinyl, pyridyl, piperazinyl, pyrrolidinyl, quinidinyl, thiazolinyl, morpholine, guanidino or amidino type, and mixtures thereof.

15. Polymer according to one of the preceding claims, in which the monomer of formula (IIa) is chosen, alone or as a mixture, from dimethylaminopropyl-(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate, and the monomers below:

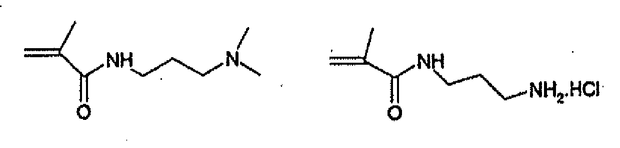

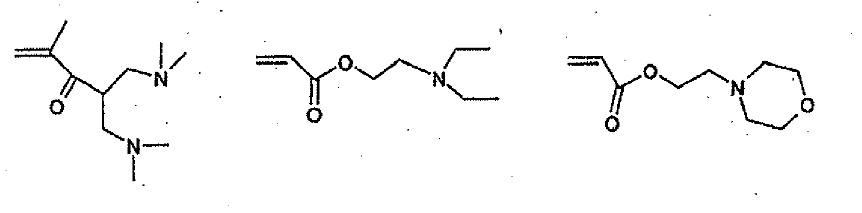

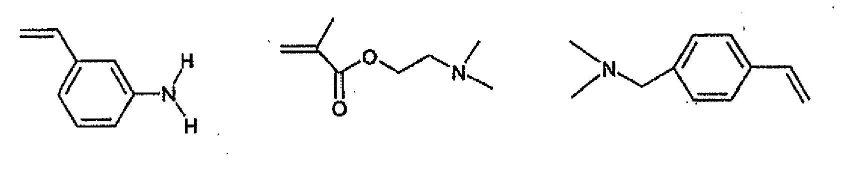

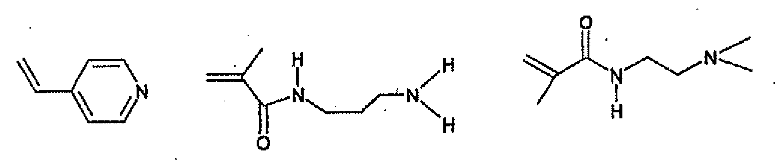

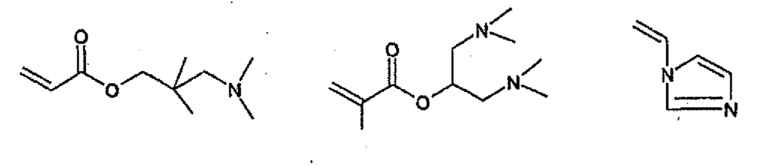

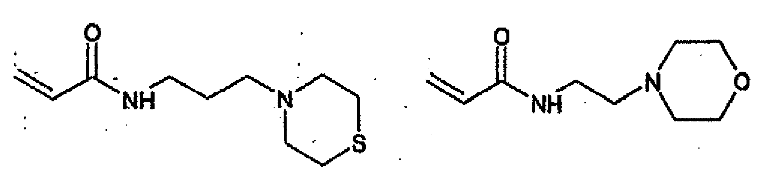

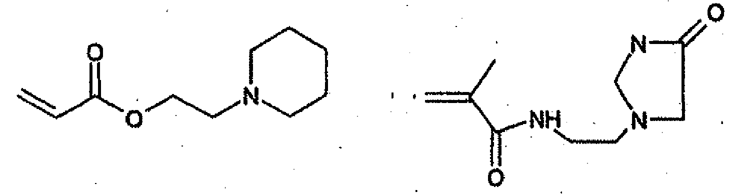

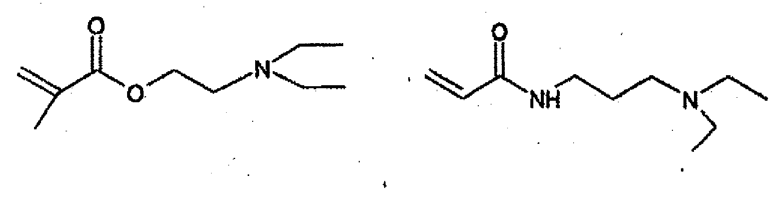

16. Polymer according to one of the preceding claims, in which the monomers of formula (IIa) are chosen, alone or as a mixture, from dimethylaminopropyl-(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate.

17. Polymer according to one of the preceding claims, in which the anionic monomers are chosen from maleic anhydride, acrylic acid, methacrylic acid, crotonic acid, itaconic acid, fumaric acid, maleic acid, 2-carboxyethyl acrylate (CH₂=CH-C (O) -O- (CH₂) ₂-COOH) ; styrenesulfonic acid, 2-acrylamido-2-methylpropanesulfonic acid, vinylbenzoic acid, vinylphosphoric acid and sulfopropyl (meth)acrylate, and salts thereof.

18. Polymer according to one of the preceding claims, in which, in formula (IIc), the radical X'⁺ is chosen from radicals such as pyridyl, indolyl, isoindolinyl, imidazolyl, imidazolinyl, piperidyl, pyrazolinyl, quinolyl, piperazinyl, pyrrolidinyl, quinidinyl, thiazolinyl, morpholinyl, guanidino and amidino, and mixtures thereof.

19. Polymer according to one of the preceding claims, in which, in formulae (IIc) and/or (IId), the radical R'3 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 5 heteroatoms chosen from 0, N, S, F, Si and P; or alternatively a benzylene radical -C₆H₄CH₂-, optionally substituted with a C1-C12 alkyl radical optionally comprising 1 to 5 heteroatoms chosen from 0, N, S, F, Si and P;
- a radical of formula -CH₂-O-CO-O-, CH₂-CH₂-O-CO-O-, -CH₂-CO-O-, -CH₂-CH₂-CO-O-, - [ (CH₂) ₅-CO-O]ₙ-, -CH₂-CH(CH₃)-O-, -(CH₂)₂-O-, -CH₂-O-CO-NH-, -CH₂-CH₂-O-CO-NH-: -CH₂-NHH-CO-NH- or -CH₂ -CH₂-NH-CO-NH-, -CH₂-CHOH-, -CH₂-CH₂-CHOH-, -CH₂-CH₂-CH (NH₂) -, -CH₂-CH (NH₂) -, -CH₂-CH₂-CH (NHR') -, -CH₂-CH(NHR') -, -CH₂-CH₂-CH (NR'R") -, -CH₂-CH (NR' R") -, -CH₂-CH₂-CH₂-NR' -, -CH₂ -CH₂-CH₂-O-; - [CH₂-CH₂-O]ₙ- and -[CH₂-CH(CH₃)-O]ₙ-, -CH₂-CH₂-CHR',-O- with R' and R" representing a linear or branched C1-C22 alkyl optionally comprising 1 to 12 heteroatoms chosen from 0, N, S, F, Si and P;
- or a mixture of these radicals.

20. Polymer according to one of the preceding claims, in which, in formula (IId), X"⁺ is chosen from trimethylammonium, triethylammonium, N,N-dimethyl-N-octylammonium and N,N-dimethyl-N-laurylammonium radicals.

21. Polymer according to one of the preceding claims, in which the monomers of formula (IIc) or (IId) are chosen from N,N-dimethyl-N-(2-methacryloyloxyethyl)-N-(3-sulfopropyl)ammonium betaine, N,N-dimethyl-N-(3-methacrylamidopropyl)-N-(3-sulfopropyl)ammonium betaine, 1-(3-sulfopropyl)-2-vinylpyridinium betaine and 2-methacryloyloxyethylphosphorylcholine.

22. Polymer according to one of the preceding claims, in which, when the "essentially cationic" monomer is chosen from mixtures of cationic and/or amphoteric monomers with anionic monomers,
said anionic monomers are preferably present in a proportion of from 5% to 40% by weight, especially from 10% to 30% by weight and preferably from 15% to 25% by weight relative to the weight of the "cationic and/or amphoteric + anionic monomers" mixture.

23. Polymer according to one of the preceding claims, in which the "essentially cationic" monomer is present in a proportion of from 45% inclusive to 80% inclusive by weight and preferably from 50% inclusive to 70% inclusive by weight relative to the weight of the final polymer.

24. Polymer according to one of the preceding claims, in which at least one of the monomers and/or the polymer is neutralized with a neutralizer chosen from mineral bases, organic bases, mineral acids and/or organic acids.

25. Polymer according to one of the preceding claims, in which at least one of the monomers and/or the polymer is neutralized with a neutralizer of mineral or organic acid type, having a logP of less than or equal to 2, for example between -8 and 2, preferably between -6 and 1 and especially between -6 and 0; or alternatively having a logP of greater than 2, preferably greater than or equal to 2.5, especially greater than 3, and in particular between 3 and 15, or even between 3.5 and 10.

26. Polymer according to one of the preceding claims, in which at least one of the monomers and/or the polymer is neutralized with a neutralizer of mineral or organic acid type, having a logP of less than or equal to 2, for example between -8 and 2, preferably between -6 and 1 and especially between -6 and 0.

27. Polymer according to one of the preceding claims, in which at least one of the monomers and/or the polymer is neutralized by a neutralizer chosen from 2-ethylcaproic acid, oleic acid, behenic acid, stearic acid, acetic acid, citric acid, tartaric acid, betaine hydrochloride and/or gluconic acid, and preferentially behenic acid and/or betaine hydrochloride.

28. Polymer according to one of the preceding claims, in which:
- the monomer of formula (I), alone or as a mixture, is present in a proportion of from 10% inclusive to 60% exclusive by weight, especially from 20% inclusive to 55% inclusive by weight and preferably from 30% inclusive to 50% inclusive by weight, relative to the weight of the final polymer, and is chosen, alone or as a mixture, from poly(ethylene glycol) (meth)acrylates and/or methylpoly(ethylene glycol) (meth)acrylates, preferably those with a molecular weight between 350 and 13 000 g/mol and especially between 500 and 8000 g/mol; and/or
- the "essentially cationic" monomer is present in a proportion of from 40% exclusive to 90% inclusive by weight, especially from 45% inclusive to 80% inclusive by weight and preferably from 50% inclusive to 70% inclusive by weight, relative to the weight of the final polymer, and is chosen, alone or as a mixture, from the monomers of formula (IIb).

29. Polymer according to one of the preceding claims, in which:
- the monomer of formula (I), alone or as a mixture, is present in a proportion of from 10% inclusive to 60% exclusive by weight, especially from 20% inclusive to 55% inclusive by weight and preferably from 30% inclusive to 50% inclusive by weight, relative to the weight of the final polymer, and is chosen, alone or as a mixture, from poly(ethylene glycol) (meth)acrylates, preferably those with a molecular weight between 350 and 13 000 g/mol and especially between 500 and 8000 g/mol; and/or
- the "essentially cationic" monomer is present in a proportion of from 40% exclusive to 900 % inclusive by weight, especially from 45% inclusive to 80% inclusive by weight and preferably from 50% inclusive to 70% inclusive by weight, relative to the weight of the final polymer, and is chosen, alone or as a mixture, from dimethylaminopropyl(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate.

30. Polymer according to one of the preceding claims, in which:
- the monomer of formula (I), alone or as a mixture, is present in a proportion of from 10% inclusive to 60% exclusive by weight, especially from 20% inclusive to 55% inclusive by weight and preferably from 30% inclusive to 50% inclusive by weight relative to the weight of the final polymer, and is chosen, alone or as a mixture, from poly(ethylene glycol) (meth)acrylates, preferably those with a molecular weight of between 350 and 13 000 g/mol and especially between 500 and 8000 g/mol; and
- the "essentially cationic" monomer is present in a proportion of from 40% exclusive to 90% inclusive by weight, especially from 45% inclusive to 80% inclusive by weight and preferably from 50% inclusive to 70% inclusive by weight relative to the weight of the final polymer and is chosen, alone or as a mixture, from dimethylaminopropyl(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate; and
- the polymer is neutralized with a neutralizer chosen from 2-ethylcaproic acid, oleic acid, behenic acid, stearic acid, acetic acid, citric acid, tartaric acid, betaine hydrochloride and/or gluconic acid, and preferentially behenic acid and/or betaine, hydrochloride.

31. Polymer according to one of the preceding claims, in which:
- the monomer of formula (I), alone or as a mixture, is present in a proportion of from 10% inclusive to 60% exclusive by weight, especially from 20% inclusive to 55% inclusive by weight and preferably from 30% inclusive to 50% inclusive by weight relative to the weight of the final polymer and is chosen, alone or as a mixture, from poly(ethylene glycol) (meth)acrylates, preferably those with a molecular weight of between 350 and 13 000 g/mol and especially between 500 and 8000 g/mol; and
- the "essentially cationic" monomer is present in a proportion of from 40% exclusive to 90% inclusive by weight, especially from 45% inclusive to 80% inclusive by weight and preferably from 50% inclusive to 70% inclusive by weight relative to the weight of the final polymer and is chosen, alone or as a mixture, from dimethylaminopropyl(meth)acrylamide, and
- the polymer is neutralized with a neutralizer chosen from behenic acid and/or betaine hydrochloride.

32. Polymer according to one of the preceding claims, with a weight-average molecular mass (Mw) of between 500 and 5 000 000, especially between 1000 and 3 000 000 and even more preferentially between 2000 and 2 000 000, or even 4000 and 500 000, inter alia between 7000 and 400 000, better still between 20 000 and 350 000 and even better between 150 000 and 300 000.

33. Polymer according to one of the preceding claims, **characterized in that** it is conveyable in aqueous medium, preferably water-soluble or water-dispersible.

34. Polymer according to one of the preceding claims, **characterized in that** it has a viscosity in water of between 1 and 1000 mPa·s, preferably between 1.5 and 750 mPa.s and better still between 2 and 500 mPa·s, measured at 25°C, using a Brookfield viscometer, for a solution containing 15% by weight of the polymer in water or methyl ethyl ketone, at 25°C, with a No. 1 spindle of needle type; for a measuring time of 5 minutes, at a speed of between 0.1 and 6 rpm.

35. Polymer according to one of the preceding claims, **characterized in that** it has a glass transition temperature (Tg) of between -150°C and 20°C, especially -120°C and 10°C and better still between -100°C and 0°C.

36. Polymer according to one of the preceding claims, **characterized in that** it has a water uptake of between 3% and 150% by weight, preferably between 4% and 100% by weight and especially between 5% and 50% by weight, at 75% relative humidity (75% PH) ; and/or **in that** it has a water uptake of between 3% and 200% by weight, preferably between 2.5% and 150% by weight, especially between 3% and 100% by weight, at 85% relative humidity (85% RH).

37. Cosmetic or pharmaceutical composition comprising, in a physiologically acceptable medium, especially a cosmetically or pharmaceutically acceptable medium, at least one polymer as defined in one of Claims 1 to 36.

38. Composition according to Claim 37, in which the polymer is present in a proportion of from 0.01% to 50% by weight, especially from 0.1% to 30% by weight or even from 0.3% to 10% by weight and better still from 1% to 3% by weight of solids relative to the total weight of the composition.

39. Composition according to either of Claims 37 and 38, in which the physiologically acceptable medium comprises at least one constituent chosen from water; hydrophilic organic solvents, for instance alcohols, especially linear or branched C1-C6 monoalcohols and polyols and glycol ethers, especially C₂ glycol ethers and hydrophilic C₂-C₄ aldehydes; waxes, pasty fatty substances, gums and mixtures thereof, of animal, plant, mineral or synthetic origin; lipophilic organic solvents; oils of animal, plant, mineral or synthetic origin; synthetic esters and ethers; pentaerythritol esters; fatty alcohols containing from 12 to 26 carbon atoms; partially hydrocarbon-based and/or silicone-based fluoro oils; volatile or nonvolatile, linear or cyclic silicone oils, which are liquid or pasty at room temperature; pigments, nacres, fillers, water-soluble dyes and liposoluble dyes; polymers; auxiliary film-forming agents; surfactants; vitamins, fragrances, nacreous agents, thickeners, gelling agents, trace elements, softeners, sequestrants, fragrances, acidifying or basifying agents, preserving agents, sunscreens, antioxidants, hair-loss counteractants, antidandruff agents, propellants and ceramides; mixtures thereof.

40. Composition according to one of Claims 37 to 39, which is in the form of a suspension, a dispersion especially of oil in water by means of vesicles; an optionally thickened or even gelled oily solution; an oil-in-water, water-in-oil or multiple emulsion; a gel or a mousse; an oily or emulsified gel; a dispersion of vesicles, especially lipid vesicles; a two-phase or multiphase lotion; a spray; a lotion, a cream, a pomade, a soft paste, an ointment, a cast or moulded solid especially in stick or dish form, or alternatively a compacted solid.

41. Composition according to one of Claims 37 to 40, which is in the form of a care and/or makeup product for bodily or facial skin, for the lips and the hair, an antisun or self-tanning product or a haircare product.

42. Composition according to one of Claims 37 to 41, which is in the form of a hair composition, especially for holding the hairstyle or shaping the hair, for example in the form of shampoos, hairsetting gels or lotions, blow-drying lotions, fixing and styling compositions such as lacquers or sprays; rinse-out or leave-in hair conditioners, compositions for permanent-waving, relaxing, dyeing or bleaching the hair, or alternatively in the form of rinse-out compositions, to be applied before or after dyeing, bleaching, permanent-waving or relaxing the hair or between the two steps of a permanent-waving or hair-relaxing operation.

43. Composition according to one of Claims 37 to 42, comprising (% by weight):
- 7.5% of lauryl ether sulfate,
- 2.5% of cocobetaine amphoteric surfactant,
- 5% of coco-polyglucoside surfactant,
- 1.5% of polymer according to the intention, and
- qs 100% water;
the said polymer comprising 50% by weight of poly(ethylene glycol) methacrylate with a molecular weight of 550 g/mol and 50% by weight of dimethylaminopropylmethacrylamide, and being neutralized with betaine hydrochloride.

44. Composition according to Claim 43, which is in the form of a shampoo.

45. Cosmetic process for treating keratin materials such as bodily or facial skin, the nails, bodily hair, head hair and/or the eyelashes, **characterized in that** it consists in applying to the keratin materials a Cosmetic composition as defined in one of Claims 37 to 44.

## Patentansprüche

1. Copolymer auf der Basis von ethylenisch ungesättigten Monomeren, das im Wesentlichen aus folgenden Bestandteilen besteht, ausgedrückt in Gewichtsprozent, bezogen auf das Gesamtgewicht des Polymers:
- a) zu 10 bis 60 Gew.-% aus mindestens einem Monomer der Formel (I) und/oder seinen Salzen: 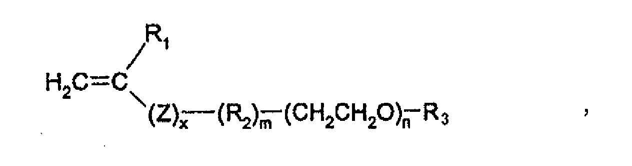 worin bedeuten:
- R₁ ein Wasserstoffatom oder eine geradkettige oder verzweigte Kohlenwasserstoffgruppe vom Typ CₚH₂ₚ₊₁, wobei p eine ganze Zahl von 1 bis einschließlich 12 bedeutet;
- Z eine zweiwertige Gruppe, die ausgewählt ist unter -COO-, -CONH-, -CONCH₃-, -OCO-, -O-, -SO₂-, -CO-O-CO- oder -CO-CH₂-CO-;
- x 0 oder 1;
- R₂ eine gesättigte oder ungesättigte, gegebenenfalls aromatische, geradkettige, verzweigte oder cyclische zweiwertige kohlenstoffhaltige Gruppe mit 1 bis 30 Kohlenstoffatomen, die 1 bis 18 Heteroatome enthalten kann, die unter O, N, S, F, Si und P ausgewählt sind;
- m 0 oder 1;
- n eine ganze Zahl von 3 bis einschließlich 300;
- R₃ ein Wasserstoffatom oder eine gesättigte oder ungesättigte, gegebenenfalls aromatische, geradkettige, verzweigte oder cyclische Kohlenstoff enthaltende Gruppe mit 1 bis 30 Kohlenstoffatomen, die 1 bis 20 Heteroatome enthalten kann, die unter O, N, S, F, Si und P ausgewählt sind,
und
- b) zu 40 bis 90 Ges.-% aus mindestens einem "im Wesentlichen kationischen" Monomer und/oder seinen Salzen, das ausgewählt ist unter:
- (i) einem oder mehreren kationischen Monomeren der Formel (IIa),
- (ii) einem oder mehreren amphoteren Monomeren der Formel (IIc) und (IId) und
- (iii) einem Gemisch von einem oder mehreren kationischen Monomeren der Formel (IIa) mit einem oder mehreren anionischen Monomeren, die unter Maleinsäureanhydrid und/oder Monomeren der Formel (IIb) ausgewählt sind, und/oder mit einem oder mehreren amphoteren Monomeren, die unter den Monomeren der Formeln (IIc) und (IId) ausgewählt sind, 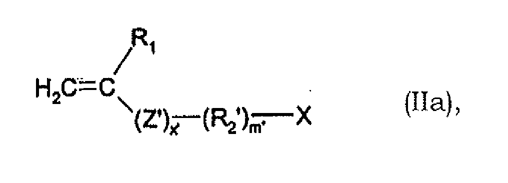 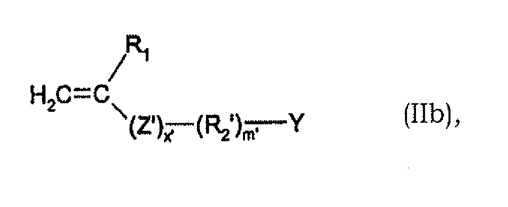 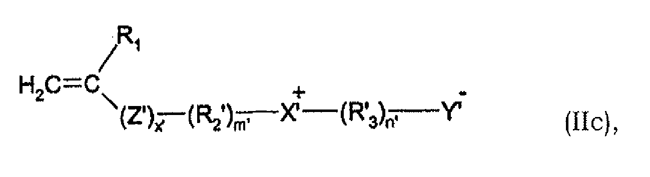 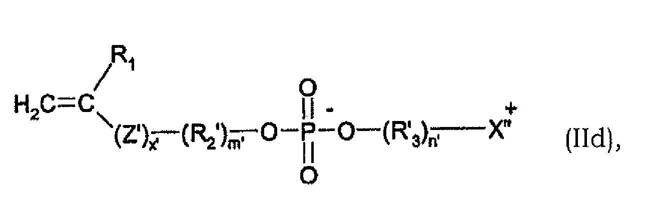
wobei in den obigen Formeln bedeuten:
- R₁ ein Wasserstoffatom oder eine geradkettige oder verzweigte Kohlenwasserstoffgruppe vom Typ CₚH₂ₚ₊₁, wobei p eine ganze Zahl von 1 bis einschließlich 12 ist, vorzugsweise Wasserstoff oder eine Gruppe Methyl, Ethyl, Propyl, Butyl;
- Z' eine zweiwertige Gruppe, die ausgewählt ist unter -COO-, -CONH-, -CONCH₃-, -OCO-, -O-, -SO₂-, -CO-O-CO- oder -CO-CH₂-CO-, wobei COO und CONH bevorzugt sind;
- x' 0 oder 1, vorzugsweise 1;
- R₂' eine gesättigte oder ungesättigte, gegebenenfalls aromatische, geradkettige, verzweigte oder cyclische zweiwertige kohlenstoffhaltige Gruppe mit 1 bis 30 Kohlenstoffatomen, die 1 bis 18 Heteroatome enthalten kann, die unter O, N, S, F, Si und P ausgewählt sind;
- m' 0 oder 1;
- X (in Formel IIa)
(a) eine Gruppe Guanidino oder Amidino oder
(b) eine Gruppe der Formel -N(R₆)(R₇) oder -P(R₆)(R₇) oder -P⁺R₆R₇R₈, wobei R₆, R₇ und R₈ unabhängig voneinander bedeuten: entweder (i) ein Wasserstoffatom oder (ii) eine geradkettige, verzweigte oder cyclische, gesättigte oder ungesättigte und gegebenenfalls aromatische Alkylgruppe mit 1 bis 18 Kohlenstoffatomen, die 1 bis 10 Heteroatome enthalten kann, die unter O, N, S, F, Si und P ausgewählt sind, oder (iii) R₆ und R₇ können mit dem Stickstoffatom oder dem Phosphoratom einen ersten, gesättigten oder ungesättigten und gegebenenfalls aromatischen Ring bilden, der insgesamt 5, 6, 7 oder 8 Kohlenstoffatome und insbesondere 4, 5 oder 6 Kohlenstoffatome und/oder 2 bis 4 Heteroatome enthält, die unter O, S und N ausgewählt sind, wobei der erste Ring mit einem oder mehreren weiteren, gesättigten oder ungesättigten und gegebenenfalls aromatischen Ringen verbunden sein kann, die jeweils 5, 6 oder 7 Kohlenstoffatome und insbesondere 4, 5, 6 oder 7 Kohlenstoffatome und/oder 2 bis 4 Heteroatome aufweisen, die unter O, S und N ausgewählt sind, oder
(c) eine Gruppe -R'₆-N-R'₇, bei der R'₆ und R'₇ mit dem Stickstoffatom einen gesättigten oder ungesättigten, gegebenenfalls aromatischen Ring bilden, der insgesamt 5, 6, 7 oder 8 Kohlenstoffatome und insbesondere 4, 5 oder 6 Kohlenstoffatome und/oder 2 bis 4 Heteroatome aufweist, die unter O, S und N ausgewählt sind, wobei dieser Ring mit einem oder mehreren weiteren gesättigten oder ungesättigten und gegebenenfalls aromatischen Ringen verbunden sein kann, die jeweils 5, 6 oder 7 Kohlenstoffatome und insbesondere 4, 5, 6, 7 oder 8 Kohlenstoffatome und/oder 2 bis 4 Heteroatome aufweisen, die unter O, S und N ausgewählt sind;
- Y eine Gruppe, die ausgewählt ist unter -COOH, -SO₃H, -OSO₃H, -PO₃H₂ und -OPO₃H₂;
- X'⁺ eine zweiwertige Gruppe der Formel -N⁺(R₆)(R₇)-, wobei R₆ und R₇ unabhängig voneinander bedeuten: entweder (i) ein Wasserstoffatom oder (ii) eine geradkettige, verzweigte oder cyclische und gegebenenfalls aromatische Alkylgruppe mit 1 bis 25 Kohlenstoffatomen, die 1 bis 20 Heteroatome aufweisen kann, die unter O, N, S und P ausgewählt sind, oder (iii) R₆ und R₇ können zusammen mit dem Stickstoffatom einen ersten gesättigten oder ungesättigten und gegebenenfalls aromatischen Ring mit insgesamt 5, 6, 7 oder 8 Kohlenstoffatomen und insbesondere 4, 5, 6 oder 7 Kohlenstoffatomen und/oder 2 bis 3 Heteroatomen bilden, die unter O, S und N ausgewählt sind, wobei der erste Ring mit einem oder mehreren weiteren gesättigten oder ungesättigten und gegebenenfalls aromatischen Ringen verbunden sein kann, die jeweils 5, 6, 7 oder 8 Kohlenstoffatome und insbesondere 4, 5, 6 oder 7 Kohlenstoffatome und/oder 2 bis 3 Heteroatome aufweisen, die unter O, S und N ausgewählt sind;
- Y'- eine Gruppe, die unter -COO-, -SO₃-. -OSO₃⁻, -PO₃²⁻ und -OPO₃²⁻ ausgewählt ist;
- R'₃ eine gesättigte oder ungesättigte, gegebenenfalls aromatische, geradkettige, verzweigte oder cyclische zweiwertige kohlenstoffhaltige Gruppe mit 1 bis 30 Kohlenstoffatomen, die 1 bis 18 Heteroatome aufweisen kann, die unter O, N, S, F, Si und P ausgewählt sind;
- n' 1 bis einschließlich 100 und vorzugsweise 1 bis einschließlich 5;
- X"⁺ eine Gruppe der Formel -N⁺R₆R₇R₈, wobei R₆, R₇ und R₈ unabhängig voneinander bedeuten: entweder (i) ein Wasserstoffatom oder (ii) eine geradkettige, verzweigte oder cyclische und gegebenenfalls aromatische Alkylgruppe mit 1 bis 18 Kohlenstoffatomen, die 1 bis 5 Heteroatome aufweisen kann, die unter O, N, S und P ausgewählt sind, oder (iii) R₆ und R₇ können zusammen mit dem Stickstoffatom einen ersten, gesättigten oder ungesättigten und gegebenenfalls aromatischen Ring mit insgesamt 5, 6 oder 7 Kohlenstoffatomen und insbesondere 4, 5 oder 6 Kohlenstoffatomen und/oder 2 bis 3 Heteroatomen bilden, die unter O, S und N ausgewählt sind, wobei der erste Ring mit einem oder mehreren weiteren gesättigten oder ungesättigten und gegebenenfalls aromatischen Ringen verbunden sein kann, die jeweils 5, 6 oder 7 Kohlenstoffatome und insbesondere 4, 5, 6 oder 7 Kohlenstoffatome und/oder 2 bis 3 Heteroatome aufweisen, die unter O, S und N ausgewählt sind,
sowie die Salze des Copolymers.

2. Polymer nach Anspruch 1, bei dem in Formel (I) R₁ Wasserstoff oder Methyl, Ethyl, Propyl oder Butyl bedeutet.

3. Polymer nach einem der vorhergehenden Ansprüche, bei dem in Formel (I) Z COO oder CONH bedeutet.

4. Polymer nach einem der vorhergehenden Ansprüche, bei dem die Gruppe R₂ in Formel (I) ausgewählt ist unter:
- einer Alkylengruppe, wie Methylen, Ethylen, Propylen, n-Butylen, Isobutylen, tert.-Butylen, n-Hexylen, n-Octylen, n-Dodecylen, n-Octadecylen, n-Tetradecylen und n-Docosanylen;
- einer Phenylengruppe -C₆H₄- (o-, m- oder p-), die gegebenenfalls mit einer C₁-C₁₂-Alkylgruppe substituiert ist, die gegebenenfalls 1 bis 25 Heteroatome aufweist, die unter O, N, S, F, Si und P ausgewählt sind, oder einer Benzylgruppe -C₆H₄-CH₂-, die gegebenenfalls mit einer C₁-C₁₂-Alkylgruppe substituiert ist, die gegebenenfalls 1 bis 8 Heteroatome aufweist, die unter O, N, S, F, Si und P ausgewählt sind;
- einer Pyridiniumgruppe der Formel 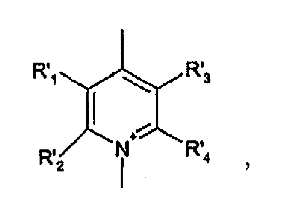 worin R'₁ bis R'₄, die gleich oder verschieden sind, ausgewählt sind unter H und einer C₁-C₁₂-Alkylgruppe, die gegebenenfalls 1 bis 8 Heteroatome aufweist, die unter O, N, S, F, Si und P ausgewählt sind, wobei R'₁ bis R'₄ insbesondere Methyl und/oder Ethyl sein können;
- einer Gruppe der Formel -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH- oder -CH₂-CH₂-NH-CO-NH- ; -CH₂-CH₂-CH₂-O-; -CH₂-CH₂-CHR'-O-, wobei R' und R" eine geradkettige oder verzweigte C₁-C₂₂-Alkylgruppe bedeuten, die gegebenenfalls 1 bis 12 Heteroatome aufweist, die unter O, N, S, F, Si und P ausgewählt sind,
- oder einem Gemisch dieser Gruppen.

5. Polymer nach einem der vorhergehenden Ansprüche, bei dem n in Formel (I) 5 bis einschließlich 200 und noch günstiger 7 bis einschließlich 100 und ganz besonders 9 bis einschließlich 50 bedeutet.

6. Polymer nach einem der vorhergehenden Ansprüche, bei dem R₃ in der Formel (I) bedeutet: ein Wasserstoffatom; Succinimido, Maleinimido, Mesityl, Tosyl, Triethoxysilan, Phthalimid oder -CH₂-CH₂CN oder auch eine Benzylgruppe oder Phenylgruppe, die gegebenenfalls mit einer C₁-C₁₂-Alkylgruppe substituiert ist, die gegebenenfalls 1 bis 8 Heteroatome aufweist, die unter O, N, S, F, Si und P ausgewählt sind, oder eine C₁-C₃₀-Alkylgruppe, insbesondere eine C₁-C₂₂-Alkylgruppe und ganz besonders eine C₂-C₁₆-Alkylgruppe, die gegebenenfalls 1 bis 18 Heteroatome aufweist, die unter O, N, S, F, Si und P ausgewählt sind,
wobei die Gruppen Benzyl, Phenyl oder Alkyl ferner eine Funktion aufweisen können, die unter den nachstehenden Funktionen ausgewählt ist: Succinimido; Glutarat-succinimido; Glutarat; Maleinimido; Mesityl; Benzoat; Tosyl; Triethoxysilan; Phthalimid; Thioester; Benzotriazolcarbonat; Butyraldehyd; Acetaldehyd-diethylacetal; Biotin; Phospholipid; Succinat; N-Hydroxysuccinimid; -SO₃H, -COOH, -PO₄, -NR₅R₆ oder -N⁺R₅R₆R₇, wobei R₅, R₆ und R₇ unabhängig voneinander ausgewählt sind unter H oder geradkettigen, verzweigten oder cyclischen C₁-C₁₈-Alkylgruppen, insbesondere Methyl, die gegebenenfalls 1 oder mehrere Heteroatome oder auch Schutzgruppen wie tert.-Butyloxycarbonyl oder 9-Fluorenylmethoxycarbonyl enthalten.

7. Polymer nach einem der vorhergehenden Ansprüche, bei dem das Monomer der Formel (I) ausgewählt ist unter folgenden Monomeren allein oder in Form von Gemischen:
- Poly(ethylenglycol)-(meth)acrylaten;
- Methyl-poly(ethylenglycol)-(meth)acrylaten;
- Alkyl-poly(ethylengylcol)-(meth)acrylaten;
- Phenyl-poly(ethylenglycol)-(meth)acrylaten;
- dem nachstehenden Monomer: 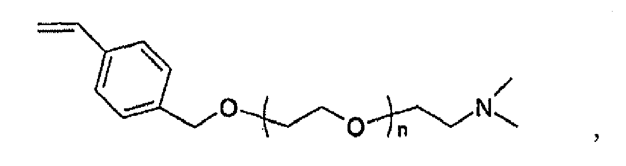 bei dem n vorzugsweise im Bereich von 3 bis einschließlich 100, insbesondere im Bereich von 5 bis einschließlich 50 und ganz besonders im Bereich von 7 bis einschließlich 30 liegt.

8. Polymer nach einem der vorhergehenden Ansprüche, bei dem das Monomer der Formel (I) ein Molekulargewicht von 350 bis 13 000 g/mol und insbesondere im Bereich von 500 bis 8000 g/mol aufweist.

9. Polymer nach einem der vorhergehenden Ansprüche, bei dem das Monomer der Formel (I) allein oder in Form eines Gemisches ausgewählt ist unter Poly(ethylenglycol)-(meth)acrylaten und Methyl-poly(ethylenglycol)-(meth)acrylaten, vorzugsweise solchen mit einem Molekulargewicht von 350 bis 13 000 g/mol und insbesondere von 500 bis 8000 g/mol.

10. Polymer nach einem der vorhergehenden Ansprüche, bei dem das Monomer der Formel (I) allein oder in Form eines Gemisches ausgewählt ist unter Poly(ethylenglycol)-(meth)acrylaten, insbesondere solchen mit einem Molekulargewicht von 350 bis 13 000 g/mol und ganz besonders von 500 bis 8000 g/mol.

11. Polymer nach einem der vorhergehenden Ansprüche, bei dem das Monomer der Formel (I) allein oder in Form eines Gemisches in einem Mengenanteil von 20 bis einschließlich 55 Gew.-%, bezogen auf das Gewicht des fertigen Polymers, und vorzugsweise in einem Mengenanteil von 30 bis einschließlich 50 Gew.-% vorliegt.

12. Polymer nach einem der vorhergehenden Ansprüche, bei dem die Gruppe R'₂ in den Formeln (IIa), (IIb), (IIc) und/oder (IId) ausgewählt ist unter:
- einer Alkylengruppe wie Methylen, Ethylen, Propylen, n-Butylen, Isobutylen, tert.-Butylen, n-Hexylen, n-Octylen, n-Dodecylen, n-Octadecylen, n-Tetradecylen und n-Docosanylen;
- einer Phenylengruppe -C₆H₄- (o-, m- oder p-), die gegebenenfalls mit einer C₁-C₁₂-Alkylgruppe substituiert ist, die gegebenenfalls 1 bis 5 Heteroatome aufweist, die unter O, N, S, F, Si und/oder P ausgewählt sind, oder einer Benzylgruppe -C₆H₄-CH₂-, die gegebenenfalls mit einer C₁-C₁₂-Alkylgruppe substituiert ist, die gegebenenfalls 1 bis 8 Heteroatome aufweist, die unter O, N, S, F, Si und P ausgewählt sind;
- einer Gruppe der Formel-CH₂-O-CO-O-, CH₂-CH₂-O-CO-O-, -CH₂-CO-O-, -CH₂-CH₂-CO-O-, -[(CH₂)₅-CO-O]n-, -CH₂-CH(CH₃)-O-, -(CH₂)₂-O-, -CH₂-O-CO-NH-, -CH₂-CH₂-O-CO-NH-; -CH₂-NH-CO-NH- oder -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O- oder -CH₂-CH₂-CHR'-O-, wobei R' und R" eine geradkettige oder verzweigte C₁-C₂₂-Alkylgruppe bedeuten, die gegebenenfalls 1 bis 12 Heteroatome aufweist, die unter O, N, S, F, Si und P ausgewählt sind,
- oder einem Gemisch dieser Gruppen.

13. Polymer nach einem der vorhergehenden Ansprüche, bei dem die Gruppen R₆ und R₇ in der Formel (IIa), die in X vorliegen, ausgewählt sind unter Wasserstoff, Methyl, Ethyl, Propyl, Isopropyl, n-Butyl, tert.-Butyl, Isobutyl, Octyl, Lauryl und Stearyl.

14. Polymer nach einem der vorhergehenden Ansprüche, bei dem X in Formel (IIa) eine Gruppe ist, die ausgewählt ist unter Gruppen vom Typ Pyridin, Indolyl, Isoindolinyl, Imidazolyl, Imidazolinyl, Piperidinyl, Pyrazolinyl, Pyrazolyl, Chinolin, Pyrazolinyl, Pyridinyl, Piperazinyl, Pyrrolidinyl, Chinidinyl; Thiazolinyl, Morpholin, Guanidino und Amidino sowie ihren Gemischen.

15. Polymer nach einem der vorhergehenden Ansprüche, bei dem das Monomer der Formel (IIa) allein oder in Form eines Gemisches ausgewählt ist unter Dimethylaminopropyl-(meth)acrylamid, Dimethylaminoethyl-(meth)acrylamid, Diethylaminoethyl-(meth)acrylat, Dimethylaminoethyl-(meth)acrylat, Vinylimidazol, Vinylpyridin und Morpholinethyl-(meth)acrylat sowie den nachstehend aufgeführten Monomeren:

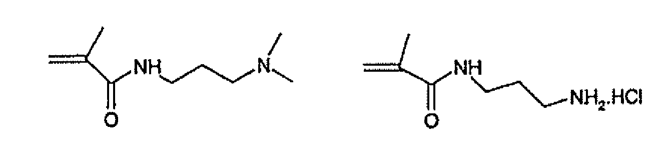

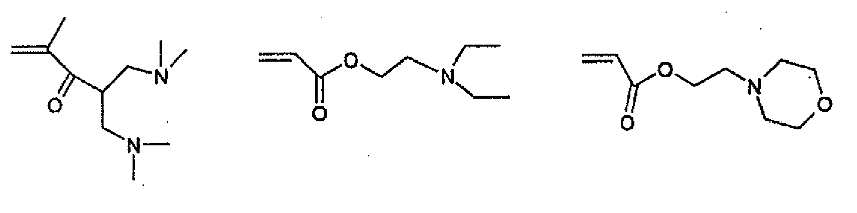

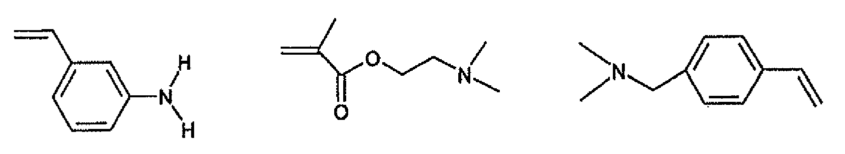

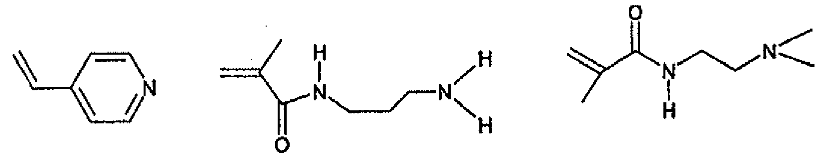

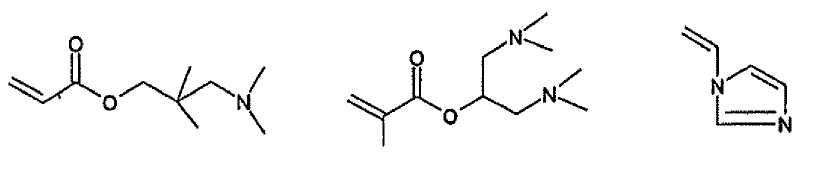

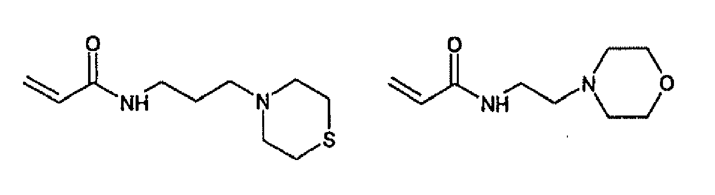

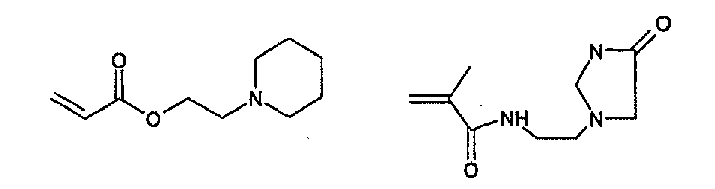

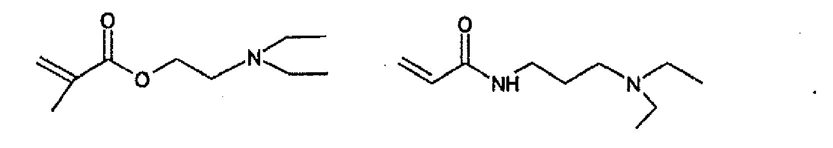

16. Polymer nach einem der vorhergehenden Ansprüche, bei dem die Monomeren der Formel (IIa) allein oder in Form eines Gemisches ausgewählt sind unter Dimethylaminopropyl-(meth)acrylamid, Dimethylaminoethyl-(meth)acrylamid, Diethylaminoethyl-(meth)acrylat, Dimethylaminoethyl-(meth)acrylat, Vinylimidazol, Vinylpyridin und Morpholinoethyl-(meth)acrylat.

17. Polymer nach einem der vorhergehenden Ansprüche, bei dem die anionischen Monomeren ausgewählt sind unter Maleinsäureanhydrid, Acrylsäure, Methacrylsäure, Crotonsäure, Itaconsäure, Fumarsäure, Maleinsäure, 2-Carboxyethylacrylat (CH₂=CH-C(O)-O-(CH₂)₂-COOH), Styrolsulfonsäure, 2-Acrylamido-2-methylpropansulfonsäure, Vinylbenzoesäure, Vinylphosphorsäure und Sulfopropyl(meth)acrylat sowie den Salzen dieser Verbindungen.

18. Polymer nach einem der vorhergehenden Ansprüche, bei dem die Gruppe X'⁺ in Formel (IIc) ausgewählt ist unter Gruppen vom Typ Pyridin, Indolyl, Isoindolinyl, Imidazolyl, Imidazolinyl, Piperidinyl, Pyrazolinyl, Pyrazolyl, Chinolin, Pyrazolinyl, Pyridinyl, Piperazinyl, Pyrrolidinyl, Chinidinyl, Thiazolinyl, Morpholin, Guanidino und Amidino sowie ihren Gemischen.

19. Polymer nach einem der vorhergehenden Ansprüche, bei dem die Gruppe R'₃ in den Formeln (IIc) und/oder (IId) ausgewählt ist unter:
- einer Alkylengruppe wie Methylen, Ethylen, Propylen, n-Butylen, Isobutylen, tert.-Butylen, n-Hexylen, n-Octylen, n-Dodecylen, n-Octadecylen, n-Tetradecylen und n-Docosanylen;
- einer Phenylengruppe -C₆H₄- (o-, m- oder p-), die gegebenenfalls mit einer C₁-C₁₂-Alkylgruppe substituiert ist, die gegebenenfalls 1 bis 5 Heteroatome aufweist, die unter O, N, S, F, Si und P ausgewählt sind; oder einer Benzylengruppe -C₆H₄-CH₂-, die gegebenenfalls mit einer C₁-C₁₂-Alkylgruppe substituiert ist, die gegebenenfalls 1 bis 5 Heteroatome aufweist, die unter O, N, S, F, Si und P ausgewählt sind;
- einer Gruppe der Formel-CH₂-O-CO-O-, CH₂-CH₂-O-CO-O-, -CH₂-CO-O-, -CH₂-CH₂-CO-O-, -[(CH₂)₅-CO-O]n-, -CH₂-CH(CH₃)-O-, -(CH₂)₂-O-, -CH₂-O-CO-NH-, -CH₂-CH₂-O-CO-NH-; -CH₂-NH-CO-NH- oder -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; [CH₂-CH₂-O]ₙ- und -[CH₂-CH(CH₃)-O]ₙ- oder -CH₂-CH₂-CHR'-O-, wobei R' und R" eine geradkettige oder verzweigte C₁-C₂₂-Alkylgruppe bedeuten, die gegebenenfalls 1 bis 12 Heteroatome aufweist, die unter O, N, S, F, Si und P ausgewählt sind,
- oder einem Gemisch dieser Gruppen.

20. Polymer nach einem der vorhergehenden Ansprüche, bei dem in der Formel (IId) X"⁺ unter den Gruppen Trimethylammonium, Triethylammonium, N,N-Dimethyl-N-octylammonium und N,N-Dimethyl-N-laurylammonium ausgewählt ist.

21. Polymer nach einem der vorhergehenden Ansprüche, bei dem das Monomer der Formel (IIc) oder (IId) ausgewählt ist unter N,N-Dimethyl-N-(2-methacryloyloxyethyl)-N-(3-sulfopropyl)-ammoniumbetain, N,N-Dimethyl-N-(3-methacrylamidopropyl)-N-(3-sulfopropyl)-ammoniumbetain, 1-(3-Sulfopropyl)-2-vinylpyridiniumbetain und 2-Methacryloyloxyethylphosphorylcholin.

22. Polymer nach einem der vorhergehenden Ansprüche, bei dem, wenn das "im Wesentlichen kationische" Monomer unter Gemischen von kationischen und/oder amphoteren Monomeren mit anionischen Monomeren ausgewählt ist, die anionischen Monomeren vorzugsweise in einem Mengenanteil von 5 bis 40 Gew.-%, bezogen auf das Gewicht des Gemisches "kationischen und/oder amphotere + anionische Monomere", vorliegen, insbesondere in einem Mengenanteil von 10 bis 30 Gew.-% und vorzugsweise von 15 bis 25 Gew.-%.

23. Polymer nach einem der vorhergehenden Ansprüche, bei dem das "im Wesentlichen kationische" Monomer in einem Mengenanteil von 45 bis einschließlich 80 Gew.-%, bezogen auf das Gewicht des fertigen Polymers, und vorzugsweise in einem Mengenanteil von 50 bis einschließlich 70 Gew.-% vorliegt.

24. Polymer nach einem der vorhergehenden Ansprüche, bei dem mindestens eines der Monomeren und/oder das Polymer mit einem Neutralisationsmittel neutralisiert ist, das unter anorganischen Basen, organischen Basen, anorganischen Säuren und/oder organischen Säuren ausgewählt ist.

25. Polymer nach einem der vorhergehenden Ansprüche, bei dem mindestens eines der Monomeren und/oder das Polymer mit einem Neutralisationsmittel vom Typ einer anorganischen oder organischen Säure neutralisiert ist, die einen Wert log P kleiner als oder gleich 2 aufweist, der beispielsweise im Bereich von -8 bis 2, vorzugsweise im Bereich von -6 bis 1 und insbesondere im Bereich von -6 bis 0 liegt, oder einen Wert log P, der größer ist als 2, vorzugsweise gleich oder größer als 2,5, insbesondere größer als 3, und der ganz besonders im Bereich von 3 bis 15, und ganz speziell im Bereich von 3,5 bis 10 liegt.

26. Polymer nach einem der vorhergehenden Ansprüche, bei dem mindestens eines der Monomeren und/oder das Polymer mit einem Neutralisationsmittel vom Typ einer anorganischen oder organischen Säure neutralisiert ist, die einen Wert log P kleiner oder gleich 2 aufweist, zum Beispiel im Bereich von -8 bis 2, vorzugsweise im Bereich von -6 bis 1 und insbesondere im Bereich von -6 bis 0.

27. Polymer nach einem der vorhergehenden Ansprüche, bei dem mindestens eines der Monomeren und/oder das Polymer mit einem Neutralisationsmittel neutralisiert ist, das ausgewählt ist unter 2-Ethylcapronsäure, Ölsäure, Behensäure, Stearinsäure, Essigsäure, Citronensäure, Weinsäure, Betainhydrochlorid und/oder Gluconsäure, vorzugsweise mit Behensäure und/oder Betainhydrochlorid.

28. Polymer nach einem der vorhergehenden Ansprüche, bei dem
- das Monomer der Formel (I) allein oder in Form eines Gemisches in einem Mengenanteil von 10 bis 60 Gew.-%, bezogen auf das Gewicht des fertigen Polymers, insbesondere in einem Mengenanteil von 20 bis 55 Gew.-% und vorzugsweise in einem Mengenanteil von 30 bis 50 Gew.-% vorliegt und allein oder in Form eines Gemisches ausgewählt ist unter Poly(ethylenglycol)-(meth)acrylaten und/oder Poly(ethylenglycol)-(meth)acrylaten, vorzugsweise solchen mit einem Molekulargewicht von 350 bis 13 000 g/mol und insbesondere im Bereich von 500 bis 8000 g/mol, und/oder
- das "im Wesentlichen kationische" Monomer in einem Mengenanteil von 40 bis 90 Gew.-%, bezogen auf das Gewicht des fertigen Polymers, insbesondere in einem Mengenanteil von 45 bis 80 Gew.-% und bevorzugt in einem Mengenanteil von 50 bis 70 Gew.-% vorliegt und allein oder in Form eines Gemisches unter den Monomeren der Formel (IIb) ausgewählt ist.

29. Polymer nach einem der vorhergehenden Ansprüche, bei dem
- das Monomer der Formel (I) allein oder in Form eines Gemisches in einem Mengenanteil von 10 bis 60 Gew.-%, bezogen auf das Gewicht des fertigen Polymers, insbesondere in einem Mengenanteil von 20 bis 55 Gew.-% und vorzugsweise in einem Mengenanteil von 30 bis 50 Gew.-% vorliegt und allein oder im Gemisch unter Poly(ethylenglycol)-(meth)acrylaten ausgewählt ist, vorzugsweise solchen mit einem Molekulargewicht im Bereich von 350 bis 13 000 g/mol und insbesondere im Bereich von 500 bis 8000 g/mol, und
- das "im Wesentlichen kationische" Monomer in einem Mengenanteil von 40 bis 90 Gew.-%, bezogen auf das Gewicht des fertigen Polymers, insbesondere in einem Mengenanteil von 45 bis 80 Gew.-% und bevorzugt in einem Mengenanteil von 50 bis 70 Gew.-% vorliegt und allein oder in Form eines Gemisches ausgewählt ist unter Dimethylaminopropyl-(meth)acrylamid, Dimethylaminoethyl-(meth)acrylamid, Diethylaminoethyl-(meth)acrylat, Dimethylaminoethyl-(meth)acrylat, Vinylimidazol, Vinylpyridin und Marpholinoethyl-(meth)acrylat.

30. Polymer nach einem der vorhergehenden Ansprüche, bei dem
- das Monomer der Formel (I) allein oder in Form eines Gemisches in einem Mengenanteil von 10 bis 60 Gew.-%, bezogen auf das Gewicht des fertigen Polymers, insbesondere in einem Mengenanteil von 20 bis 55 Gew.-% und vorzugsweise in einem Mengenanteil von 30 bis 50 Gew.-% vorliegt und allein oder in Form eines Gemisches unter Poly(ethylenglycol)-(meth)acrylaten ausgewählt ist, vorzugsweise solchen mit einem Molekulargewicht im Bereich von 350 bis 13 000 g/mol und insbesondere im Bereich von 500 bis 8000 g/mol, und
- das "im Wesentlichen kationische" Monomer in einem Mengenanteil von 40 bis 90 Gew.-%, bezogen auf das Gewicht des fertigen Polymers, insbesondere in einem Mengenanteil von 45 bis 80 Gew.-% und bevorzugt in einem Mengenanteil von 50 bis 70 Gew.-% vorliegt und das allein oder in Form eines Gemisches ausgewählt ist unter Dimethylaminopropyl-(meth)acrylamid, Dimethylaminoethyl-(meth)acrylamid, Diethylaminoethyl-(meth)acrylat, Dimethylaminoethyl-(meth)acrylat, Vinylimidazol, Vinylpyridin und Morpholinoethyl-(meth)acrylat und
- das Polymer mit einem Neutralisationsmittel neutralisiert ist, das ausgewählt ist unter 2-Ethylcapronsäure, Ölsäure, Behensäure, Stearinsäure, Essigsäure, Citronensäure, Weinsäure, Betainhydrochlorid und/oder Gluconsäure, vorzugsweise mit Behensäure und/oder Betainhydrochlorid.

31. Polymer nach einem der vorhergehenden Ansprüche, bei dem
- das Monomer der Formel (I) allein oder in Form eines Gemisches in einem Mengenanteil von 10 bis 60 Gew.-%, bezogen auf das Gewicht des fertigen Polymers, insbesondere in einem Mengenanteil von 20 bis 55 Gew.-% und vorzugsweise in einem Mengenanteil von 30 bis 50 Gew.-% vorliegt und das allein oder in Form eines Gemisches unter Poly(ethylenglycol)-(meth)acrylaten ausgewählt ist, vorzugsweise solchen mit einem Molekulargewicht im Bereich von 350 bis 13 000 g/mol und insbesondere im Bereich von 500 bis 8000 g/ mol, und
- das "im Wesentlichen kationische" Monomer in einem Mengenanteil von 40 bis 90 Gew.-%, bezogen auf das Gewicht des fertigen Polymers, insbesondere in einem Mengenanteil von 45 bis 80 Gew.-% und bevorzugt in einem Mengenanteil von 50 bis 70 Gew.-% vorliegt und das allein oder in Form eines Gemisches unter Dimethylaminopropyl-(meth)acrylamid ausgewählt ist und
- das Polymer mit einem Neutralisationsmittel neutralisiert ist, das unter Behensäure und/oder Betainhydrochlorid ausgewählt ist.

32. Polymer nach einem der vorhergehenden Ansprüche, das ein Gewichtsmittel des Molekulargewichts (Mw) im Bereich von 500 bis 5 000 000 aufweist, insbesondere im Bereich von 1000 bis 3 000 000, noch bevorzugter im Bereich von 2000 bis 2 000 000, besonders im Bereich von 4000 bis 500 000, ganz besonders im Bereich von 7000 bis 400 000, günstiger im Bereich von 20 000 bis 350 000 und noch günstiger im Bereich von 150 000 bis 300 000.

33. Polymer nach einem der vorhergehenden Ansprüche, **dadurch gekennzeichnet, dass** es in ein wässeriges Medium einbringbar und vorzugsweise wasserlöslich oder in Wasser dispergierbar ist.

34. Polymer nach einem der vorhergehenden Ansprüche, **dadurch gekennzeichnet, dass** es eine Viskosität in Wasser von 1 bis 1 000 mPa·s, vorzugsweise von 1,5 bis 750 mPa·s und noch günstiger von 2 bis 500 mPa·s aufweist, gemessen bei 25 °C mit einem BROOKFIELD-Viskosimeter an einer Lösung mit einem Gehalt von 15 Gew.-% des Polymers in Wasser oder Methylethylketon bei 25 °C mit einem beweglichen Teil vom Nadeltyp (Spindel) Nr. 1 während einer Messzeit von 5 Minuten bei einer Geschwindigkeit von 0,1 bis 6 Umdrehungen/Minute.

35. Polymer nach einem der vorhergehenden Ansprüche, **dadurch gekennzeichnet, dass** es eine Glasübergangstemperatur (Tg) im Bereich von - 50 bis 20 °C, insbesondere im Bereich von -120 bis 10 °C und noch günstiger im Bereich von -100 bis 0 °C aufweist.

36. Polymer nach einem der vorhergehenden Ansprüche, **dadurch gekennzeichnet, dass** es eine Wasseraufnahme von 3 bis 150 Gew.-%, vorzugsweise von 4 bis 100 Gew.-% und insbesondere von 5 bis 50 Gew.-% bei einer relativen Feuchte von 75 % (75 % RF) aufweist und/oder eine Wasseraufnahme von 3 bis 200 Gew.-%, vorzugsweise von 2,5 bis 150 Gew.-% und insbesondere von 3 bis 100 Gew.-% bei einer relativen Feuchte von 85 % (85 % RF) aufweist.

37. Kosmetische oder pharmazeutische Zusammensetzung, die in einem physiologisch akzeptablen Medium, insbesondere einem kosmetisch oder pharmazeutisch akzeptablen Medium, mindestens ein Polymer enthält, wie es in einem der Ansprüche 1 bis 36 definiert ist.

38. Zusammensetzung nach Anspruch 37, in der das Polymer in einem Mengenanteil von 0,01 bis 50 Gew.-%, bezogen auf die Trockensubstanz, insbesondere von 0,1 bis 30 Gew.-%, günstiger von 0,3 bis 10 Gew.-% und noch günstiger von 1 bis 3 Gew.-% vorliegt, bezogen auf das Gesamtgewicht der Zusammensetzung.

39. Zusammensetzung nach Anspruch 37 oder 38, bei der das physiologisch akzeptable Medium mindestens einen Bestandteil enthält, der ausgewählt ist unter Wasser; hydrophilen organischen Lösungsmitteln wie Alkoholen, insbesondere einwertigen, geradkettigen oder verzweigten C₁-C₆-Alkoholen und Polyolen und Glycolethern, insbesondere C₂-Glycolethern, und hydrophilen C₂-C₄-Aldehyden; Wachsen, pastosen Fettstoffen, Gummen und ihren Gemischen, tierischen, pflanzlichen, mineralischen oder synthetischen Ursprungs; lipophilen organischen Lösungsmitteln; tierischen Ölen, Pflanzenölen, Mineralölen oder synthetischen Ölen; synthetischen Estern und Ethern; Pentaerythritestern; Fettalkoholen mit 12 bis 26 Kohlenstoffatomen; partiell fluorierten Kohlenwasserstoffen und/oder Siliconölen; flüchtigen oder nichtflüchtigen, geradkettigen oder cyclischen, bei Umgebungstemperatur flüssigen oder pastosen Siliconölen; Pigmenten, Perlglanzstoffen, Füllstoffen; wasserlöslichen Farbstoffen, öllöslichen Farbstoffen; Polymeren; Hilfsstoffen zur Filmbildung; Tensiden, Vitaminen, Parfums, Perlglanzmitteln, Verdickungsmitteln, Gelbildnern, Spurenelementen, reizlindernden Mitteln, Sequestrierungsmitteln, Parfums, Mitteln zum Alkalischmachen oder zum Ansäuern, Konservierungsmitteln, Sonnenfiltern, Antioxidationsmitteln, Mitteln gegen Haarausfall, Mitteln gegen Schuppen, Treibmitteln und Ceramiden sowie Gemischen dieser Stoffe.

40. Zusammensetzung nach einem der Ansprüche 37 bis 39, die vorliegt in Form
einer Suspension, einer Dispersion, insbesondere einer Öl-in-Wasser-Dispersion aufgrund von Vesikeln; einer Öllösung, die gegebenenfalls verdickt und insbesondere in Gelform übergeführt ist; einer Öl-in-Wasser-Emulsion, einer Wasser-in-Öl-Emulsion oder einer multiplen Emulsion; eines Gels oder eines Schaums; eines öligen oder emulgierten Gels, einer Dispersion von Vesikeln, insbesondere von Lipidvesikeln; einer zweiphasigen oder multiphasigen Lotion; eines Sprays; einer Lotion, einer Creme, einer Pomade, einer weichen Paste, einer Salbe, eines gegossenen oder geformten Feststoffs und insbesondere in Form eines Sticks oder eines Feststoffs in Schälchen oder in Form eines kompaktierten Feststoffs.

41. Zusammensetzung nach einem der Ansprüche 37 bis 40, die in Form eines zur Pflege und/oder zum Schminken der Haut, des Körpers oder des Gesichts, der Lippen und der Haare, in Form eines Sonnenschutzprodukts oder eines Selbstbräunungsprodukts oder in Form eines Produkts zur Haarpflege vorliegt.

42. Zusammensetzung nach einem der Ansprüche 37 bis 41, die vorliegt in Form
- einer Zusammensetzung zur Haarpflege, insbesondere zum Haltbarmachen der Frisur oder zum Frisieren der Haare, zum Beispiel in Form von Shampoos, Gelen, Lotionen zum Legen, Lotionen für eine Fönfrisur, Festigerzusammensetzungen und Frisierzusammensetzungen wie Haarlacke oder Haarsprays; Apres-Shampoos zum Ausspülen oder Après-Shampoos, die nicht ausgespült werden müssen, Zusammensetzungen für Dauerwellen, Zusammensetzungen zum Entkräuseln, Zusammensetzungen zum Färben oder zum Entfärben oder auch in Form von Zusammensetzungen zum Spülen, die vor oder nach einem Färben, einem Entfärben, einer Dauerwelle oder einem Entkräuseln oder auch zwischen den beiden Stufen einer Dauerwelle oder eines Entkräuselns anzuwenden ist.

43. Zusammensetzung nach einem der Ansprüche 37 bis 42, die enthält (in Gew.-%):
- 7,5 % Laurylethersulfat
- 2,5 % amphoteres Cocobetain-Tensid
- 5 % Coco-Polyglucosid-Tensid
- 1,5 % Polymer gemäß der Erfindung und
- Wasser q.s.p. 100 %,
wobei das Polymer 50 Gew.-% Poly(ethylenglycol)-(meth)acrylat mit einem Molekulargewicht von 550 g/mol und 50 Gew.-% Dimethylaminopropylmethacrylamid enthält und mit Betainhydrochlorid neutralisiert ist.

44. Zusammensetzung nach Anspruch 43, die in Form eines Shampoos vorliegt.

45. Kosmetisches Verfahren zur Behandlung von Keratinmaterialien wie der Haut des Körpers oder des Gesichts, der Nägel, des Körperhaars, der Haare und/oder der Wimpern, **dadurch gekennzeichnet, dass** es darin besteht, auf die Keratznmaterialien eine kosmetische Zusammensetzung aufzubringen, wie sie in einem der Ansprüche 37 bis 44 definiert ist.
